(19) Europäisches Patentamt
European Patent Office
Office européen des brevets

(11) **EP 3 957 241 B1**

(12) **EUROPÄISCHE PATENTSCHRIFT**

(45) Veröffentlichungstag und Bekanntmachung des Hinweises auf die Patenterteilung:
**25.01.2023 Patentblatt 2023/04**

(21) Anmeldenummer: **21189030.6**

(22) Anmeldetag: **02.08.2021**

(51) Internationale Patentklassifikation (IPC):
*A61B 5/0536* (2021.01) *A61B 5/085* (2006.01)
*A61M 16/00* (2006.01)

(52) Gemeinsame Patentklassifikation (CPC):
**A61B 5/0536; A61B 5/085; A61M 16/024;**
A61M 16/01; A61M 2016/0027; A61M 2016/0036;
A61M 2205/3344; A61M 2205/502; A61M 2209/084;
A61M 2210/1039; A61M 2210/105;
A61M 2210/1053; A61M 2230/04; A61M 2230/46;
A61M 2230/60; (Forts.)

(54) **ANORDNUNG UND COMPUTERPROGRAMMPRODUKT ZUR ERMITTLUNG DER REGIONALEN LUNGEN-COMPLIANCE**

ASSEMBLY AND COMPUTER PROGRAM PRODUCT FOR DETERMINING REGIONAL LUNG COMPLIANCE

AGENCEMENT ET PRODUIT PROGRAMME D'ORDINATEUR DE DÉTERMINATION DE LA COMPLIANCE PULMONAIRE PAR ZONES

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **07.08.2020 DE 102020120900**

(43) Veröffentlichungstag der Anmeldung:
**23.02.2022 Patentblatt 2022/08**

(73) Patentinhaber: **Drägerwerk AG & Co. KGaA**
**23558 Lübeck (DE)**

(72) Erfinder:
• **Teschner, Eckhard**
**23558 Lübeck (DE)**
• **Ralfs, Frank**
**23558 Lübeck (DE)**

(56) Entgegenhaltungen:
**WO-A1-82/01654 US-A1- 2002 133 081**

• **EDUARDO L V COSTA ET AL: "Bedside estimation of recruitable alveolar collapse and hyperdistension by electrical impedance tomography", INTENSIVE CARE MEDICINE, SPRINGER, BERLIN, DE, Bd. 35, Nr. 6, 3. März 2009 (2009-03-03), Seiten 1132-1137, XP019699473, ISSN: 1432-1238**

(52) Gemeinsame Patentklassifikation (CPC): (Forts.)
A61M 2230/65

**Beschreibung**

Die Erfindung betrifft eine Anordnung und ein Computerprogrammprodukt zur Ermittlung eines Maßes für die jeweilige regionale Dehnbarkeit der Lunge eines Patienten in mehreren unterschiedlichen Bereichen der Lunge. Die Dehnbarkeit wird auch als "Compliance" bezeichnet.

Im Folgenden wird die Bezeichnung "respiratorisches System" des Patienten verwendet. Das respiratorische System umfasst dessen Lunge und dessen Brustkorb.

Wenn ein Patient spontan atmet und / oder künstlich beatmet wird, so sollen folgende beiden unerwünschten Situationen vermieden werden, die jede für sich die Lunge des Patienten schädigen können:

- Der auf die Lunge ausgeübte pneumatische Druck ist zu niedrig, und die Lunge kollabiert daher (fällt in sich zusammen).
- Der ausgeübte Druck ist zu groß, und die Lunge wird überdehnt.

Ein Ansatz, um ein Beatmungsgerät abhängig von der Dehnbarkeit der Lunge zu steuern, wird beschrieben in Zhanqi Zhao u. a.: "Positive End-Expiratory Pressure Titration with Electrical Impedance Tomography and Pressure-Volume Curve in Severe Acute Respiratory Distress Syndrome", Annals of Intensive Care 9, Nr. 1 (Dezember 2019), verfügbar unter https://doi.org/10.1186/s13613-019-0484-0, abgerufen am 12.06.2020. Weitere Ansätze, um mechanische / pneumatische Eigenschaften der Lunge eines Patienten zu ermitteln und / oder um ein Beatmungsgerät zu steuern, werden beschrieben in

- Ola Stenqvist, Per Persson, and Stefan Lundin: "Can We Estimate Transpulmonary Pressure without an Esophageal Balloon?-Yes", Annals of Translational Medicine 6, Nr. 19 (Oktober 2018): 392 ff., verfügbar unter https://doi.org/10.21037/atm.2018.06.05, abgerufen am 12.06.2020,
- Giacomo Bellani u. a.: "Plateau and Driving Pressure in the Presence of Spontaneous Breathing", Intensive Care Medicine 45, Nr. 1 (Januar 2019): 97-98, verfügbar unter https://doi.org/10.1007/s00134-018-5311-9, abgerufen am 12.06.2020,
- Daniel Talmor u. a.: "Mechanical Ventilation Guided by Esophageal Pressure in Acute Lung Injury", New England Journal of Medicine 359, Nr. 20 (13. November 2008): 2095-2104, verfügbar unter https://doi.org/10.1056/NEJMoa0708638, abgerufen am 12.06.2020,
- Jeremy R. Beitler u. a.: "Effect of Titrating Positive End-Expiratory Pressure (PEEP) With an Esophageal Pressure-Guided Strategy vs. an Empirical High PEEP-FIO2 Strategy on Death and Days Free From Mechanical Ventilation Among Patients With Acute Respiratory Distress Syndrome: A Randomized Clinical Trial", JAMA, 18. Februar 2019, verfügbar unter https://doi.org/10.1001/jama.2019.0555, abgerufen am 12.06.2020.

Die Dehnbarkeit der Lunge kann sich von Bereich zu Bereich der Lunge unterscheiden. Beispielsweise aus DE 10 2005 031 752 B4 ist bekannt, verschiedene Bereiche der Lunge mithilfe von Impedanztomografie zu vermessen, bevorzugt mithilfe eines sogenannten EIT-Gürtels, der um den Körper des Patienten gelegt wird.

In Eduardo L. V. Costa u. a.: "Bedside Estimation of Recruitable Alveolar Collapse and Hyperdistension by Electrical Impedance Tomography", Intensive Care Medicine 35, Nr. 6 (Juni 2009): 1132-37, verfügbar unter https://doi.org/10.1007/s00134-009-1447-y, abgerufen am 12.06.2020, wird vorgeschlagen, die Dehnbarkeit oder Elastizität (Compliance) des respiratorischen Systems eines Patienten zu analysieren, während ein veränderlicher endexspiratorischer Druck (PEEP) eingestellt wird. Probeweise werden verschiedene Werte eingestellt, und untersucht wird, in welchen Bereichen die Compliance des respiratorischen Systems in welchem Ausmaß abnimmt, wobei diese Abnahme als eine Überdehnung oder als ein Kollaps der Lunge interpretiert wird.

Der Erfindung liegt die Aufgabe zugrunde, eine Anordnung und ein Computerprogrammprodukt bereitzustellen, welche die Dehnbarkeit der Lunge besser als bekannte Anordnungen und Computerprogrammprodukte zu messen vermögen.

Die Aufgabe wird durch eine Anordnung mit den Merkmalen des Anspruchs 1 und durch ein Computerprogrammprodukt mit den Merkmalen des Anspruchs 8 gelöst. Vorteilhafte Ausgestaltungen sind in den Unteransprüchen angegeben. Vorteilhafte Ausgestaltungen der erfindungsgemäßen Anordnung sind, soweit sinnvoll, auch vorteilhafte Ausgestaltungen des Verfahrens und umgekehrt.

Die Erfindung betrifft eine Anordnung und ein Verfahren, welche automatisch ein Maß für die jeweilige regionale Dehnbarkeit der Lunge eines Patienten in mindestens zwei unterschiedlichen Bereichen der Lunge zu ermitteln vermögen. Die regionale Dehnbarkeit ist die Dehnbarkeit der Lunge in einem Lungenbereich, wobei jeder Lungenbereich vorgegeben ist und jeweils einen Teil der Lunge, aber nicht die gesamte Lunge umfasst. Bevorzugt sind die Lungenbereiche disjunkt zueinander, überlappen sich also nicht. Jeder Bereich der Lunge, dessen regionale Dehnbarkeit ermittelt werden soll, ist so festgelegt, dass zu einem Zeitpunkt die regionale Dehnbarkeit im gesamten Lungenbereich mit einer für die Anwendung ausreichenden Genauigkeit als überall gleich angesehen wird. Die ermittelte regionale Dehnbarkeit eines Lungenbereichs kann mit der Zeit variieren, beispielsweise weil der Zustand des Patienten sich ändert oder aufgrund unterschiedlicher Einstellungen eines Beatmungsgeräts, welches den Patienten künstlich beatmet. Zu einem Zeitpunkt kann die jeweils ermittelte regionale Dehnbarkeit von Lungenbereich zu Lungenbereich variieren. Die erfindungsgemäße Anordnung umfasst eine EIT-Messvorrichtung. Diese EIT-Messvorrichtung vermag ein Maß für die Volumenänderung eines Lungenbereichs zu messen und hierfür ein Verfahren der Elektroimpedanztomographie (EIT) anzuwenden. Dank der EIT-Messvorrichtung lässt sich ein Maß für die jeweilige Volumenänderung jedes Lungenbereichs messen.

Weiterhin umfasst die erfindungsgemäße Anordnung einen Atemwegsdruck-Sensor. Dieser Atemwegsdruck-Sensor vermag ein Maß für den zeitlich veränderlichen Druck am oder im Atemweg des Patienten zu messen, bevorzugt ein pneumatisches Maß. Dieser Druck resultiert aus der eigenen Atmungsaktivität des Patienten und / oder einer künstlichen Beatmung des Patienten, die von einem Beatmungsgerät durchgeführt wird. Die eigene Atmungsaktivität des Patienten kann aus einer spontanen Atmung des Patienten sowie einer von außen stimulierten eigenen Atmungsaktivität resultieren.

Ein datenverarbeitendes Steuergerät der erfindungsgemäßen Anordnung vermag Signale von der EIT-Messvorrichtung und von dem Atemwegsdruck-Sensor zu empfangen und zu verarbeiten und berechnet für mindestens zwei unterschiedliche Bereiche der Lunge jeweils ein Maß für die regionale Dehnbarkeit der Lunge in diesem Bereich. Bevorzugt berechnet das Steuergerät das jeweilige Maß für die regionale Dehnbarkeit für vier Lungenbereiche, die nach Art von vier Quadranten angeordnet sind, oder sogar für acht Lungenbereiche.

Als dieses Maß für die regionale Dehnbarkeit eines Lungenbereichs berechnet das Steuergerät einen Quotienten aus

- einer Volumen-Differenz für diesen Lungenbereich und
- einer Druck-Differenz, die an der Lunge des Patienten anliegt.

Als Volumen-Differenz für einen Bereich der Lunge ermittelt das Steuergerät ein Maß für die Differenz zwischen dem endinspiratorischen und dem endexspiratorischen Volumen dieses Bereichs der Lunge. Das endinspiratorische Volumen tritt am Ende eines Einatmungsvorgangs des Patienten auf, das endexspiratorische Volumen am Ende eines Ausatmungsvorgangs. Bekanntlich dehnt sich die Lunge beim Einatmen und zieht sich beim Ausatmen wieder zusammen, sodass das endinspiratorische Volumen größer als das endexspiratorische Volumen ist. Um diese beiden Volumina zu ermitteln, verwendet das Steuergerät Signale der EIT-Messvorrichtung. Selbstverständlich kann die Volumen-Differenz von Lungenbereich zu Lungenbereich differieren und auch zeitlich veränderlich sein.

Als Druck-Differenz ermittelt das Steuergerät ein Maß für die Differenz zwischen dem endinspiratorischen transpulmonalen Druck und dem endexspiratorischen transpulmonalen Druck des gesamten respiratorischen Systems des Patienten. Der Begriff "transpulmonaler Druck" bezeichnet einen Druck, der von innen oder auch von außen auf die Lunge wirkt. Dieser transpulmonale Druck ist die Differenz aus

- dem Druck, der auf das gesamte respiratorische System des Patienten wirkt, und
- dem Druck auf den Pleuraspalt des Patienten.

Dieser Pleuraspalt umgibt die Lunge des Patienten. Das respiratorische System umfasst die Lunge und den Brustkorb des Patienten, und der Pleuraspalt tritt zwischen der Lunge und dem Brustkorb auf.

Das Steuergerät verwendet für die Ermittlung der Druck-Differenz Signale des Atemwegsdruck-Sensors. Diese Signale sind ein Maß für den gesamten Druck, der auf das respiratorische System einwirkt und der

- nur von der eigenen Atmungsaktivität, also von der spontanen Atmung oder einer externen stimulierten eigenen Atmungsaktivität des Patienten,
- nur von der künstlichen Beatmung des Patienten durch ein Beatmungsgerät oder
- von einer Überlagerung der eigenen Atmungsaktivität und der künstlichen Beatmung

erzeugt wird.

Die Kenntnis, wie dehnbar verschiedene Bereiche der Lunge eines Patienten sind, lässt sich dafür verwenden, um ein Beatmungsgerät gut für die künstliche Beatmung dieses Patienten einzustellen. Das Beatmungsgerät führt abhängig von mindestens einem Betriebsparameter eine Abfolge von Beatmungshüben aus. Zu den am Beatmungsgerät einstellbaren Betriebsparametern gehören beispielsweise

- der endexspiratorische Druck (PEEP),
- der endinspiratorische Druck,
- ein gewünschtes Lungenvolumen (Tidalvolumen), das ist das Volumen des Gases, welches dem Patienten bei einem Beatmungshub des Beatmungsgeräts zugeführt wird, oder

- ein gewünschter Volumenfluss zwischen dem Beatmungsgerät und dem Patienten.

Das Beatmungsgerät soll insbesondere so eingestellt werden, dass bei einer künstlichen Beatmung einerseits eine Überdehnung der Lunge vermieden wird und andererseits ausgeschlossen wird, dass die Lunge in sich zusammenfällt. Die Kenntnis, wie dehnbar die Lunge eines Patienten ist, lässt sich auch für einen Patienten-Monitor und / oder für die automatische Überwachung des Patienten verwenden. Der Patienten-Monitor empfängt und verwendet Messwerte von der erfindungsgemäßen Anordnung und zeigt bevorzugt die empfangenen Messwerten in einer von einem Menschen wahrnehmbaren Form an, insbesondere visuell auf einer Anzeigeeinheit.
Die Dehnbarkeit eines pneumatischen Systems mit veränderbarem Volumen ist bekanntlich der Quotient aus

- einer auf das System einwirkenden Druck-Differenz (Nenner des Quotienten) und
- der Volumen-Änderung, die aus dieser Druck-Differenz resultiert (Zähler des Quotienten).

Erfindungsgemäß werden eine Druck-Differenz und eine bewirkte Volumen-Änderung ermittelt.
Falls ein Patient ausschließlich durch künstliche Beatmung mit der benötigten Atemluft versorgt wird, also vollständig narkotisiert ist, liefert der aktuelle Signalwert des Sensors für den Atemwegsdruck ein gutes Maß für den Druck und damit für die Belastung, welche aktuell auf die Lunge einwirken. Häufig wird ein Patient jedoch nur so kurz wie möglich ausschließlich durch künstliche Beatmung mit Atemluft versorgt. Solange wie möglich führt der Patient zusätzlich zur künstlichen Beatmung oder auch anstelle einer künstlichen Beatmung eine eigene Atmungsaktivität, nämlich eine spontane Atmung und / oder eine stimulierte Atmung, durch, also eine Atmung mit seiner eigenen Atmungsmuskulatur. Die künstliche Beatmung unterstützt also die eigene Atmungsaktivität. In diesem Falle reicht der aktuelle Signalwert des Atemwegsdruck-Sensors allein nicht aus, um den aktuell auf die Lunge einwirkenden Druck und somit auch die einwirkende Druck-Differenz ausreichend zuverlässig zu ermitteln. Erfindungsgemäß werden vielmehr der endinspiratorische transpulmonale Druck und der endexspiratorische transpulmonale Druck ermittelt, und als Druck-Differenz wird die Differenz zwischen diesen beiden transpulmonalen Drücken verwendet.
Die bewirkte Volumen-Änderung der gesamten Lunge lässt sich mithilfe eines Volumen-Sensors oder eines Volumenfluss-Sensors ermitteln. Beispielsweise wird mehrmals hintereinander der aktuelle Volumenfluss in die oder aus der Lunge des Patienten gemessen, und über die Messwerte wird aufintegriert. Jedoch kann die Dehnbarkeit der Lunge sich von Lungenbereich zu Lungenbereich erheblich unterscheiden, weswegen eine globale Volumen-Änderung der Lunge in vielen Fällen nicht ausreicht, um eine regionale Dehnbarkeit zu bestimmen. Daher wird erfindungsgemäß die jeweilige Dehnbarkeit von mindestens zwei verschiedenen Lungenbereichen ermittelt, bevorzugt von vier Lungenbereichen, die wie vier Quadranten angeordnet sind, oder sogar von acht verschiedenen Lungenbereichen. Erfindungsgemäß werden hierfür Signale der EIT-Messvorrichtung verwendet. Diese Signale liefern für verschiedene Lungenbereiche jeweils ein Maß für die regionale Volumen-Änderung des Lungenbereichs aufgrund der Atmung / Beatmung. Bevorzugt wird für jeden Bereich der Lunge oder wenigstens für jeden Bereich eines relevanten Teils der Lunge jeweils eine bewirkte Volumen-Änderung ermittelt.
Die erfindungsgemäße Anordnung und das Verfahren lassen sich selbstverständlich auch in einem Zeitraum verwenden, in dem der Patient ausschließlich durch künstliche Beatmung mit Atemluft versorgt wird. Möglich, aber dank der Erfindung nicht erforderlich ist es, beim Ermitteln der regionalen Dehnbarkeiten zwischen zwei unterschiedlichen Modi umzuschalten, nämlich

- einem Modus für die ausschließlich künstliche Beatmung und
- einem Modus für eine Überlagerung von künstlicher Beatmung und eigener Atmungsaktivität, also für die unterstützende künstliche Beatmung.

Bevorzugt befindet der Atemwegsdruck-Sensor sich außerhalb des Körpers des Patienten. Beispielsweise befindet sich ein Bestandteil des Sensors vor dem Mund des Patienten und zweigt Luft aus einem Atemluftstrom oder einem Beatmungskreislauf ab. Eine Auswerteeinheit des Sensors befindet sich räumlich entfernt vom Patienten, beispielsweise an oder in einem Beatmungsgerät oder einem Patienten-Monitor. Dank dieser Ausgestaltung ist es nicht erforderlich, einen Bestandteil des Atemwegsdruck-Sensors zeitweise in den Körper des Patienten zu verbringen und später wieder zu entnehmen. Für die Erfindung lässt sich aber auch ein Atemwegsdruck-Sensor mit einem Messwertaufnehmer im Körper des Patienten verwenden.
Der Druck im Pleuraspalt lässt sich bei einem Menschen und bei vielen anderen Lebewesen nicht direkt messen. Die Anordnung umfasst zusätzlich einen pneumatisch arbeitenden Sensor für den Druck in der Speiseröhre (esophagus). Dieser Sensor liefert ein Maß für den Druck in der Speiseröhre des Patienten. Der gemessene Druck in der Speiseröhre ist eine gute Näherung für den Druck im Pleuraspalt. Wie bereits dargelegt, ist die Differenz zwischen dem Atemwegsdruck und dem nicht direkt messbaren Druck im Pleuraspalt ein Maß für den transpulmonalen Druck. Gemäß der Erfindung wird als transpulmonaler Druck die Differenz aus

- dem Atemwegsdruck, den der Atemwegsdruck-Sensor misst, und
- dem Speiseröhren-Druck, den der Speiseröhrendruck-Sensor misst, verwendet. Um die Druck-Differenz zu ermitteln, wird mindestens einmal ein endinspiratorischer transpulmonaler Druck als Differenz zwischen dem endinspiratorischen Atemwegsdruck und dem endinspiratorischen SpeiseröhrenDruck sowie mindestens einmal ein endexspiratorischer transpulmonaler Druck als Differenz zwischen dem endexspiratorischen Atemwegsdruck und dem endexspiratorischen Speiseröhren-Druck verwendet. Bevorzugt wird an jedem Abtast-Zeitpunkt der beiden Sensoren erneut die Druck-Differenz ermittelt.

In einer bevorzugten Realisierung umfasst dieser Speiseröhrendruck-Sensor einen Katheter und einen Messballon, die beide in der Speiseröhre des Patienten positioniert sind. Der Messballon steht in einer Fluidverbindung mit dem Katheter, und der Katheter steht in einer Fluidverbindung mit einer Auswerteeinheit außerhalb des Patienten.

In einer bevorzugten Ausgestaltung wird die Erfindung verwendet, während der Patient von einem Beatmungsgerät künstlich beatmet wird, und zwar unterstützend oder ausschließlich. Das Beatmungsgerät kann als ein Anästhesiegerät ausgestaltet sein und zusätzlich den Patienten mittels mindestens eines Narkosemittels narkotisieren. Die künstliche Beatmung wird dergestalt durchgeführt, dass der endexspiratorische Druck (PEEP), den das Beatmungsgerät bewirken soll, auf einen bestimmten vorgegebenen Wert eingestellt wird. Am Ende eines Ausatmungsvorgangs soll im respiratorischen System des Patienten dieser endexspiratorische Druck herrschen. Der Atemwegsdruck-Sensor vermag den tatsächlichen endexspiratorischen Druck zu messen, sodass eine Regelung der künstlichen Beatmung mit dem Ziel, dass der tatsächliche Wert für den endexspiratorischen Druck gleich dem vorgegebenen Wert ist, möglich ist, auch eine automatische Regelung. Bevorzugt werden nacheinander mindestens zwei, bevorzugt mehr als zwei unterschiedliche Werte für den zu bewirkenden endexspiratorischen Druck vorgeben, und das Beatmungsgerät wird auf den jeweiligen Wert eingeregelt. Ein Verfahren ist nicht Teil der beanspruchten Erfindung.

In einer Fortbildung dieser Ausgestaltung gibt das Steuergerät automatisch einen ersten geforderten Wert und mindestens einen zweiten geforderten Wert für den endexspiratorischen Druck vor. Die künstliche Beatmung wird mit dem Regelungsziel durchgeführt, dass der tatsächliche Wert für den endexspiratorischen Druck zuerst gleich dem ersten vorgegebenen Wert und anschließend gleich dem oder einem zweiten vorgegeben Wert ist, wobei diese beiden Werte sich voneinander unterscheiden. Möglich ist, mehr als zwei Werte für den endexspiratorischen Druck vorzugeben und die künstliche Beatmung nacheinander auf jeden dieser mindestens drei Werte einzustellen.

Die erfindungsgemäß ermittelte regionale Dehnbarkeit eines Bereichs der Lunge hängt von dem eingestellten Wert für den endexspiratorischen Druck ab. Für jeden der mindestens zwei Werte des endexspiratorischen Drucks und für mehrere Bereiche wird gemäß dieser Ausgestaltung die jeweilige regionale Dehnbarkeit der Lunge erfindungsgemäß ermittelt.

In einer Anwendung dieser Ausgestaltung wird eine Fluidverbindung zwischen dem Beatmungsgerät und dem Patienten verwendet, um einen Soll-Betriebswert für den endexspiratorischen Druck, den das Beatmungsgerät bei der künstlichen Beatmung bewirken soll, automatisch zu ermitteln. Die jeweilige regionale Dehnbarkeit der Lunge, die bei den unterschiedlichen Werten des endexspiratorischen Drucks bewirkt wird, wird ermittelt. Über diese bewirkten regionalen Dehnbarkeiten wird geeignet kumuliert. Bevorzugt wird der geforderte Betriebswert für den endexspiratorischen Druck abhängig von einem Maß für die Dehnbarkeit der Lunge berechnen. Dieses Maß für die Lungen-Dehnbarkeit wird berechnet, indem über die erfindungsgemäß berechneten regionalen Dehnbarkeit der Lungen-Bereiche kumuliert wird.

In einer Realisierung wird für mehrere Bereiche der Lunge jeweils derjenige Wert für den exspiratorischen Druck ermittelt, der zu der höchsten regionalen Dehnbarkeit dieses Lungenbereichs führt. Dieser Optimalwert variiert in der Regel von Lungenbereich zu Lungenbereich. Für jeden berücksichtigten Lungenbereich und für mehrere Werte des endexspiratorischen Drucks wird jeweils eine relative Dehnbarkeit oder Steifigkeit oder Elastance des Lungenbereichs berechnet. Der Optimalwert für einen Lungenbereich wird abhängig von den ermittelten relativen Dehnbarkeiten automatisch festgelegt. Dieser relative Wert beträgt 100 % für den Optimalwert, der zu der maximalen regionalen Dehnbarkeit führt. Anschließend wird über die Lungenbereiche, deren regionale Dehnbarkeit ermittelt werden, kumuliert.

Die gerade beschriebene Ausgestaltung, bei der die künstliche Beatmung nacheinander auf unterschiedliche Werte für den endexspiratorischen Druck eingestellt wird, lässt sich mit der oben beschriebenen Ausgestaltung kombinieren, bei welcher der transpulmonale Druck als Differenz aus dem Atemwegsdruck und dem Speiseröhren-Druck ermittelt wird.

In einer abweichenden Ausgestaltung wird die Notwendigkeit vermieden, den Patienten mit einem pneumatischen Sensor für den Druck in der Speiseröhre auszustatten. Diese alternative Ausgestaltung lässt sich ebenfalls dann anwenden, wenn der Patient künstlich beatmet wird und der erzielte endexspiratorische Druck nacheinander auf mindestens zwei unterschiedliche Werte eingestellt wird. Jeder Wert für den endexspiratorischen Druck führt zu jeweils einem resultierenden endexspiratorischen Lungenvolumen, also dem Lungenvolumen am Ende eines Ausatmungsvorgangs. Die Differenz aus den beiden endexspiratorischen Lungenvolumina wird berechnet. Außerdem wird die Differenz zwischen den beiden Werten für den endexspiratorischen Druck berechnet. Der Quotient aus der Volumina-Differenz und der Druck-Differenz liefert ein Maß für die Differenz zwischen dem endinspiratorischen transpulmonalen Druck und dem endexspiratorischen transpulmonalen Druck.

Der Atemwegsdruck-Sensor und das Steuergerät können Bestandteile eines Beatmungsgeräts oder eines Anästhesiegeräts sein. Zwischen dem Patienten und dem Beatmungsgerät bzw. Anästhesiegerät ist wenigstens zeitweise eine Fluidverbindung, optional ein Beatmungskreislauf, hergestellt. Die EIT-Messvorrichtung steht in einer Datenverbindung mit dem Beatmungsgerät bzw. Anästhesiegerät.

Die Erfindung betrifft weiterhin ein System, welches einen Patienten künstlich zu beatmen vermag. Das System umfasst ein Beatmungsgerät, das als ein Anästhesiegerät ausgelegt sein kann, sowie eine erfindungsgemäße Anordnung. Das Beatmungsgerät vermag eine künstliche Beatmung des Patienten durchzuführen und führt bevorzugt bei der künstlichen Beatmung eine Abfolge von Beatmungshüben aus. Für diese künstliche Beatmung verwendet das Beatmungsgerät die erfindungsgemäß ermittelten regionalen Dehnbarkeiten der Lunge des Patienten. Bevorzugte Ausgestaltungen der erfindungsgemäßen Anordnung sind auch bevorzugte Ausgestaltungen dieses Systems.

Bevorzugt generiert das Beatmungsgerät jeweils einen Wert für mindestens einen Betriebsparameter des Beatmungsgeräts abhängig von den regionalen Dehnbarkeiten. Der Betriebsparameter ist beispielsweise

- das Tidalvolumen, also das Volumen der bei einem Beatmungshub zum Körper des Patienten geförderten Atem luft,
- der maximale Volumenflusses pro Minute,
- der maximale Beatmungsdruck bei einem Beatmungshub,
- der Druck am Ende eines Einatemvorgangs und vor dem nachfolgenden Ausatemvorgang, oft als Plateaudruck bezeichnet,
- der endexspiratorische Druck (PEEP), also der Druck, den die Lunge am Ende eines Beatmungshubs aufweist,
- die Rampenzeit, also die Zeit, die bei einem Beatmungshub verstreicht, bis der Beatmungsdruck den maximalen Wert erreicht hat,
- die Dauer einer Inspirationsphase, also einer Phase, bei der Atemluft zum Patienten fließt,
- die Dauer einer Exspirationsphase, also die Dauer einer Phase, bei der Atemluft vom Patienten weg fließt, oder
- die Frequenz, mit der Beatmungshübe ausgeführt werden.

Bevorzugt wird eine vorgegebene Rechenvorschrift angewendet, um den Wert des Betriebsparameters abhängig von den regionalen Dehnbarkeiten zu berechnen. Die Berechnungsvorschrift ist so vorgegeben, dass weder ein Bereich der Lunge überdehnt wird noch aufgrund eines zu geringen Drucks in sich zusammenfällt. In einer Ausgestaltung gibt das Beatmungsgerät den berechneten Wert aus, und ein Benutzer kann den Wert bestätigen oder mit einem anderen Wert überschreiben. In einer anderen Ausgestaltung verwendet das Beatmungsgerät den automatisch berechneten Wert.

Im Folgenden wird die Erfindung anhand eines Ausführungsbeispiels beschrieben. Hierbei zeigt

Figur 1     einen Patient und ein Beatmungsgerät, welches den Patienten künstlich beatmet;

Figur 2     eine schematische Darstellung von Sensoren auf einer Anzeigeeinheit des Beatmungsgeräts;

Figur 3     die zeitlichen Verläufe von mehreren Vitalparametern des Patienten;

Figur 4     eine Ausgabe von drei Werten des transpulmonalen Drucks;

Figur 5     eine beispielhafte EIT-Messvorrichtung;

Figur 6     ein Manöver, bei dem der endexspiratorische Druck schrittweise verringert wird, sowie die jeweils resultierende regionale Dehnbarkeit der Lunge.

Die Erfindung wird im Ausführungsbeispiel mithilfe einer Anordnung realisiert, welche

- ein Beatmungsgerät,
- eine EIT-Messvorrichtung,
- weitere Sensoren und
- ein übergeordnetes datenverarbeitendes Steuergerät

umfasst. Das übergeordnete Steuergerät empfängt Signale von der EIT-Messvorrichtung und den weiteren Sensoren und steuert das Beatmungsgerät abhängig von diesen Signalen an, insbesondere ein Stellglied des Beatmungsgeräts, welches die Beatmungshübe ausführt.

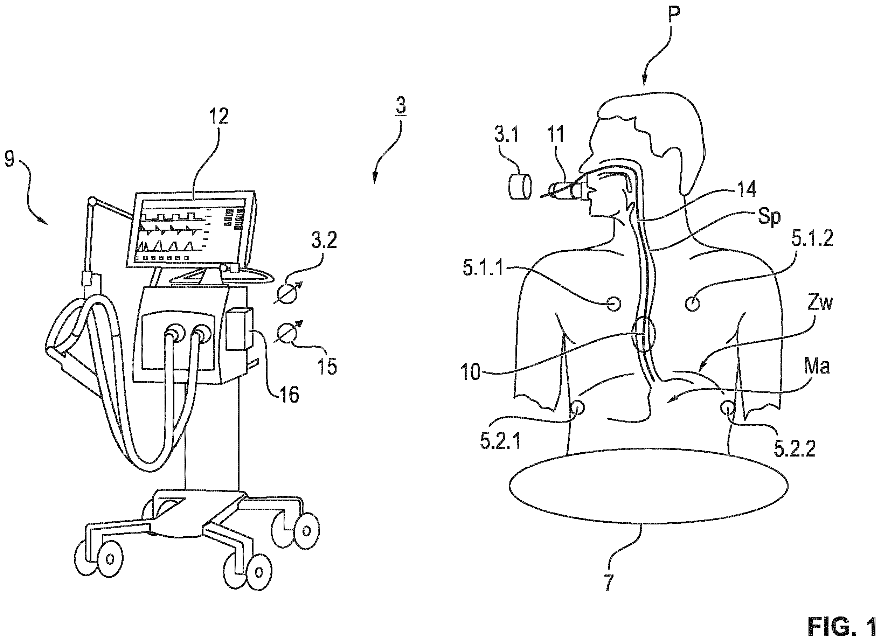

Figur 1 zeigt schematisch einen Patienten P, der künstlich beatmet wird. Gezeigt werden die Speiseröhre Sp, der Magen Ma und das Zwerchfell Zw des Patienten P. Außerdem werden

- ein Beatmungsgerät 9,
- ein flexibler Messkatheter 14,
- ein Anschlussstück 11 im Mund des Patienten P,
- ein EIT-Gürtel 7,
- mehrere beispielhafte Sensoren und
- ein übergeordnetes Steuergerät 16

gezeigt, die nachfolgend beschrieben werden.

Eine nicht gezeigte Fluidverbindung verbindet den Patienten P mit dem Beatmungsgerät 9. Durch diese Fluidverbindung kann ein Gas oder Gasgemisch von dem Beatmungsgerät 9 zu dem Patienten P fließen. Optional ist zwischen dem Patienten P und dem Beatmungsgerät 9 ein Beatmungskreislauf hergestellt, d.h. Atemluft, die der Patient P ausgeatmet hat, fließt zurück zum Beatmungsgerät 9.

Verschiedene Sensoren messen unterschiedliche Vitalparameter des Patienten P. Ein pneumatischer Sensor 3 umfasst einen Messwertaufnehmer 3.1 umfassend eine Öffnung, die in der Nähe des Mundes des Patienten P angeordnet ist und Luft aus der Fluidverbindung zwischen dem Patienten P und dem Beatmungsgerät 9 abzweigt. Die abgezweigte Luft wird über einen Schlauch an einen Drucksensor 3.2 übermittelt, der ein Maß für den Atemwegsdruck $P_{aw}$ (pressure in airway) misst. In einer Ausgestaltung ist der Messwertaufnehmer 3.1 in oder an einem Y-Stück nahe dem Anschlussstück 11 angeordnet. Optional misst ein Sensor 15 am Beatmungsgerät 9 ein Maß für das Volumen Vol' pro Zeiteinheit des Flusses von Atemluft vom Beatmungsgerät 9 zum Patienten P oder zurück vom Patienten P zum Beatmungsgerät 9. Durch Auswertung von Messwerten des Sensors 3 und / oder des Sensors 15 vermag das Beatmungsgerät 9 festzustellen, wann ein Einatmungsvorgang (Inspiration) des Patienten P beginnt und wann er endet und wann ein Ausatmungsvorgang (Exspiration) beginnt und wann er endet.

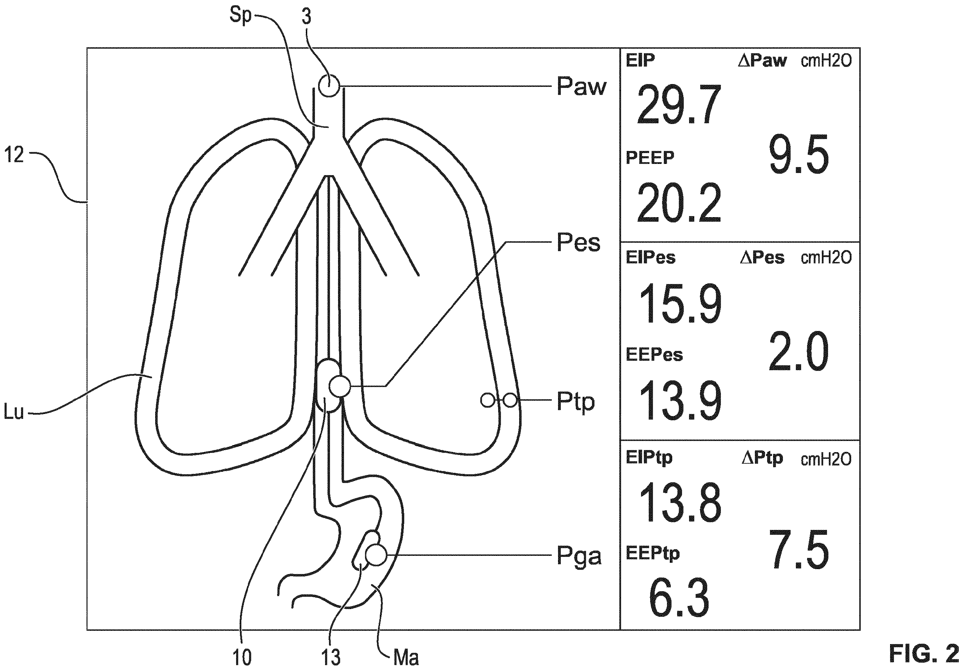

In die Speiseröhre Sp des Patienten P ist der Messkatheter 14 gelegt. Der Messkatheter 14 beginnt in dem Anschlussstück 11. Eine Sonde 10 in der Speiseröhre Sp, bevorzugt umfassend einen Messballon, misst ein Maß für den zeitlich veränderlichen pneumatischen Druck $P_{es}$ (pressure in esophagus) in der Speiseröhre Sp. Die Sonde 10 steht über den Messkatheter 14 in einer Fluidverbindung mit dem Anschlussstück 11 oder ist ein Bestandteil des Messkatheters 14. In einer Ausgestaltung ist die Sonde 10 am Übergang zwischen der Speiseröhre Sp und dem Magen Ma positioniert und umfasst einen Messballon, optional zwei Messballons. Der oder ein Messballon der Sonde 10 befindet sich im unteren Bereich der Speiseröhre Sp und verformt sich abhängig vom Druck $P_{es}$ in der Speiseröhre Sp. Der optionale andere Messballon befindet sich hinter dem Mageneingang im Magen Ma und verformt sich abhängig von dem gastralen Druck $P_{ga}$ im Magen Ma. Möglich ist auch, dass eine zusätzliche gastrale Sonde 13 in Form eines Messballons in den Magen Ma gelegt ist, vgl. Figur 2. In beiden Fällen lässt sich ein Maß für den gastralen Druck $P_{ga}$ im Magen Ma messen.

Außerdem sind bevorzugt auf der Brust des Patienten P mehrere Messelektroden befestigt. Figur 1 zeigt beispielhaft ein herznahes Paar 5.1.1, 5.1.2 von Messelektroden sowie ein zwerchfellnahes Paar 5.2.1, 5.2.2 von Messelektroden. Außerdem ist auf die Brust des Patienten P eine nicht gezeigte Messelektrode für Masse befestigt. Mit Hilfe dieser optionalen Messelektroden 5.1.1, ..., 5.2.2, Masseelektrode wird ein Elektrokardiogramm (EKG) und / oder ein Elektromyogramm (EMG) des Patienten P erzeugt. Optional werden das EKG und / oder das EMG während der künstlichen Beatmung in einer von einem Menschen wahrnehmbaren Form ausgegeben. Für das nachfolgend beschriebene Verfahren werden die Messelektroden 5.1.1 bis 5.2.2 nicht notwendigerweise benötigt.

Außerdem ist um den Körper des Patienten P ein Gürtel 7 für eine Elektroimpedanztomographie (EIT) gelegt, was weiter unten beschrieben wird. Dieser EIT-Gürtel 7 gehört zu einer EIT-Messvorrichtung, die im Ausführungsbeispiel einen weiteren Sensor für den Atemwegsdruck $P_{aw}$ (nicht gezeigt) umfasst.

Der Patient P wird von dem Beatmungsgerät 9 wenigstens zeitweise künstlich beatmet. Zwischen dem Patienten P und dem Beatmungsgerät 9 ist eine Fluidverbindung, optional ein Beatmungskreislauf, hergestellt. Das Beatmungsgerät 9 führt Beatmungshübe aus und fördert dadurch Atemluft durch die Fluidverbindung zu dem Mundstück 3 und in die Lunge des Patienten P.

Optional ist diese Atemluft mit mindestens einem Narkosemittel versehen, sodass der Patient P wenigstens teilweise narkotisiert ist.

Auf einer Anzeigeeinheit 12, die mit dem Beatmungsgerät 9 in einer Datenverbindung steht, werden

- eine schematische Darstellung der Lunge Lu und der Speiseröhre Sp des Patienten P,
- schematisch die jeweilige Position, an der ein Vitalparameter des Patienten P gemessen wird, und
- aktuelle Werte von verschiedenen Vitalparametern des Patienten P und von weiteren Parametern bei der künstlichen Beatmung des Patienten P

angezeigt. Figur 2 veranschaulicht beispielhaft eine Darstellung der Werte von verschiedenen zeitlich veränderlichen Vitalparametern auf der Anzeigeeinheit 12. Hierbei bedeuten

- $P_{aw}$ (airway pressure) ein Maß für die Atemwegsdruck,
- $P_{es}$ (pressure in esophagus, esophageal pressure) ein Maß für den Druck in der Speiseröhre Sp,
- $P_{ga}$ (gastric pressure) ein Maß für den gastralen Druck im Magen Ma,
- $P_{tp}$ (transpulmonary pressure) ein Maß für den transpulmonalen Druck, der weiter unten erläutert wird,
- EIP der aktuelle Wert für den endinspiratorischen Druck am Atemweg des Patienten P, nämlich für den Druck $P_{aw}$ am Ende eines Einatmungsvorgangs und vor dem nächsten Ausatmungsvorgang, wobei dieser endinspiratorische Druck oft auch als Plateaudruck $P_{plat}$ bezeichnet wird,
- PEEP (positive end-expiratory pressure) der aktuelle Wert für den endexspiratorischen Druck am Atemweg, nämlich für den Druck $P_{aw}$ am Ende eines Ausatmungsvorgangs und vor dem nächsten Einatmungsvorgang des Patienten P,
- ΔPaw die Differenz zwischen den aktuellen Werten für den endinspiratorischen Druck EIP und für den endexspiratorischen Druck PEEP am Atemweg, also ΔPaw = EIP - PEEP, wobei diese Differenz auch als driving pressure bezeichnet wird,
- EIPes (end-inspiratory esophageal pressure) der aktuelle Wert für den endinspiratorischen Druck in der Speiseröhre Sp, nämlich für den Druck $P_{es}$ am Ende eines Einatmungsvorgangs und vor dem nächsten Ausatmungsvorgang des Patienten P,
- EEPes (end-exspiratory esophageal pressure) der aktuelle Wert für den endexspiratorischen Druck in der Speiseröhre Sp, nämlich für den Druck $P_{es}$ am Ende eines Ausatmungsvorgangs und vor dem nächsten Einatmungsvorgang des Patienten P,
- ΔPes die Differenz zwischen den aktuellen Werten für den endinspiratorischen Druck EIPes und den endexspiratorischen Druck EEPes in der Speiseröhre Sp, also ΔPes = EIPes - EEPes,
- EIPtp (end-inspiratory transpulmonary pressure) der aktuelle Wert für den endinspiratorischen transpulmonalen Druck, nämlich für den transpulmonalen Druck $P_{tp}$ am Ende eines Einatmungsvorgangs und vor dem nächsten Ausatmungsvorgang des Patienten P,
- EEPtp (end-expiratory transpulmonary pressure) der aktuelle Wert für den endexspiratorischen transpulmonalen Druck, das ist der Druck $P_{tp}$ am Ende eines Ausatmungsvorgangs und vor dem nächsten Einatmungsvorgang des Patienten P,
- ΔPtp (transpulmonary driving pressure) die Differenz zwischen den aktuellen Werten für den endinspiratorischen transpulmonalen Druck EIPtp und den endexspiratorischen transpulmonalen Druck EEPtp, also ΔPtp = EIPtp - EEPtp,
- EEPga (end-expiratory gastric pressure, nicht gezeigt) der aktuelle Wert für den exspiratorischen gastralen Druck, das ist der gastrale Druck $P_{ga}$ am Ende eines Ausatmungsvorgangs und vor dem nächsten Einatmungsvorgang des Patienten P,
- $P_{di}$ (transdiaphragmatic pressure, ebenfalls nicht gezeigt) der aktuelle Wert für den Druck am Zwerchfell Zw als Differenz zwischen den aktuellen Werten für den gastralen Druck $P_{ga}$ und den Druck EEPes in der Speiseröhre Sp am Ende eines Ausatmungsvorgangs, also $P_{di}$ = $P_{ga}$ - EEPes.

Den Atemwegsdruck $P_{aw}$ misst der pneumatische Sensor 3, der in einer Ausgestaltung im Y-Stück vor dem Anschlussstück 11 angeordnet ist. Die Sonde 10 in der Speiseröhre Sp misst den Druck $P_{es}$ in der Speiseröhre Sp. Beispielsweise wird die Verformung oder Volumenänderung des Ballons, der zu der Sonde 10 gehört, gemessen und fungiert als ein Maß für den Druck $P_{es}$ in der Speiseröhre Sp. Ein weiterer Messballon der Sonde 10 oder eine zusätzliche gastrale Sonde 13 ermöglichen es, ein Maß für den Druck $P_{ga}$ im Magen Ma zu messen. Beispielsweise wird die Verformung oder Volumenänderung des einen Messballons, der zu der Sonde 10 gehört, gemessen und fungiert als ein Maß für den Druck $P_{es}$ in der Speiseröhre Sp. Die Verformung oder Volumenänderung des anderen Messballons der Sonde 10 oder die Verformung oder Volumenänderung der gesonderten gastralen Sonde 13 liefert ein Maß für den gastralen Druck $P_{ga}$. Die übrigen Signale werden aus Messwerten dieser Sensoren hergeleitet, was weiter unten beschrieben wird.

Figur 2 zeigt weiterhin eine schematische Darstellung der Lunge Lu, des Magens Ma und der Speiseröhre Sp des Patienten P sowie die Positionen des pneumatischen Sensors 3 und der Sonden 10 und 13.

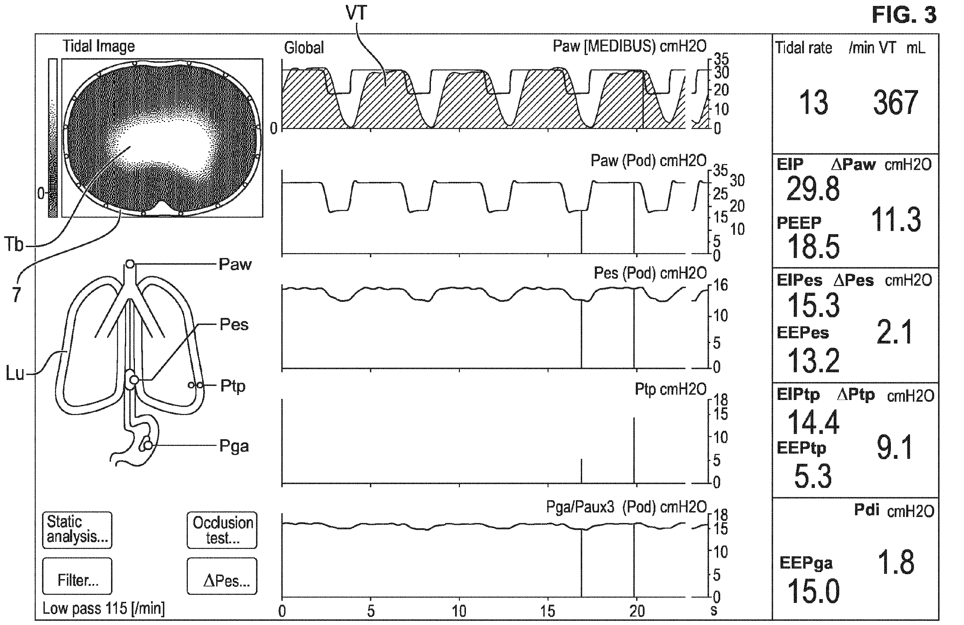

Figur 3 zeigt beispielhaft eine Darstellung mit zeitlichen Verläufe für folgende Vitalparameter:

- links oben das aktuelle Tidalbild ("tidal image") Tb, das ist die aktuelle Verteilung von Atemluft in der Lunge Lu des Patienten P, und zwar in einer horizontalen Querschnittsebene, in der sich der EIT-Gürtel 7 erstreckt, sowie eine Veranschaulichung des EIT-Gürtels 7 um die Brust oder den Magen Ma des Patienten P,
- die globale Variation eines elektrischen Widerstands (Impedanz), die mit Hilfe des EIT-Gürtels 7 gemessen wurde und die mit dem Tidalvolumen (tidal rate VT) korreliert, sowie den zeitlichen Verlauf des Atemwegsdrucks $P_{aw}$, gemessen vom pneumatischen Sensor der EIT-Messvorrichtung, in einer Darstellung in der obersten Reihe,
- den Atemwegsdruck $P_{aw}$, gemessen mit Hilfe des pneumatischen Sensors 3 (zweite Reihe von oben),
- den Druck $P_{es}$ in der Speiseröhre Sp,

- den transpulmonale Druck $P_{tp}$, bevorzugt berechnet als Differenz $P_{aw}$ - $P_{es}$,
- den gastralen Druck $P_{ga}$ im Magen Ma.

Außerdem werden die Messpositionen (links unten) sowie die aktuellen Werte von mehreren Vitalparametern (rechte Spalte) dargestellt. Die Darstellung von Figur 3 wird beispielsweise auf der Anzeigeeinheit 12 ausgegeben.

Verschiedene Betriebsparameter bei der künstlichen Beatmung des Patienten P lassen sich am Beatmungsgerät 9 einstellen, beispielsweise

- der durch einen Beatmungshub bewirkte endinspiratorische Druck EIP am Atemweg,
- ein Sollwert für den endexspiratorischen Druck PEEP,
- ein Sollwert für den Plateaudruck,
- eine untere und / oder eine obere Schranke für den Atemwegsdruck $P_{aw}$,
- das durch einen Beatmungshub erzielte Tidalvolumen VT, also die bewirkte Vergrößerung des Lungenvolumens, oder
- den Beginn, die Dauer, die Amplitude und / oder die Frequenz von Beatmungshüben, beispielsweise abhängig von der eigenen Atmungsaktivität des Patienten P.

Bevorzugt führt das Beatmungsgerät 9 eine Regelung durch, wobei ein Volumen oder ein Druck in der Fluidverbindung vorgegeben wird und als eine Führungsgröße des Regelkreises (hier: des Beatmungskreislaufs) fungiert. Die Führungsgröße kann zeitlich veränderlich sein.

Wenn ein Patient P spontan atmet und / oder künstlich beatmet wird, so dehnt sich die Lunge des Patienten P und zieht sich wieder zusammen. Jeder Beatmungshub des Beatmungsgeräts 9 führt der Lunge Luft zu und bewirkt eine Dehnung der Lunge. Das Ausatmen bewirkt, dass Luft aus der Lunge fließt. Dieses Ausdehnen und Zusammenziehen der Lunge lässt sich vereinfacht mit dem Vorgang vergleichen, einen Fahrradschlauch aufzupumpen und anschließend wieder Luft aus dem Schlauch abzulassen. Bei einer erheblich geschädigten oder erkrankten Lunge kann das Ausatmen dazu führen, dass die Lunge in sich zusammenfällt.

Zwei für den Patienten P kritische Situationen müssen vermieden werden, weil jede dieser Situationen seine Lunge beschädigen kann:

- Aufgrund eines zu geringen Drucks fällt die Lunge in sich zusammen (sie kollabiert).
- Aufgrund eines zu großen Drucks wird die Lunge überdehnt.

Insbesondere bei einer geschädigten Lunge können beide Situationen gleichzeitig auftreten, nämlich in unterschiedlichen Bereichen der Lunge, die in unterschiedlichem Ausmaß und / oder auf unterschiedliche Weisen geschädigt sind.

Die Lunge und die Brustwand (chestwall) des Patienten P bilden zusammen ein dehnbares respiratorisches System.

Falls der Patient P vollständig narkotisiert ist und keine spontane Atmung ausübt, so wird der Druck, welcher auf das respiratorische System des Patienten P einwirkt, ausschließlich vom Beatmungsgerät 9 ausgeübt. Der Druck $P_{aw}$, den der Sensor 10 in der Nähe des Munds des Patienten P misst, ist bei vollständiger Narkotisierung (Patient führt überhaupt keine eigene Atmungsaktivität aus) ein gutes Maß für den Druck, der auf das respiratorische System einwirkt. Jedoch ist die Situation zu berücksichtigen, dass der Patient P nicht vollständig narkotisiert ist, sondern wenigstens zeitweise während der künstlichen Beatmung spontan atmet oder seine eigene Atmungsaktivität stimuliert wird und die künstliche Beatmung sich mit der eigenen Atmungsaktivität (spontane Atmung und / oder stimulierte eigene Atmungsaktivität) des Patienten P überlagert. Die eigene Atmungsaktivität wird in vielen Fällen nicht in ausreichendem Maße allein durch den gemessenen Druck $P_{aw}$ berücksichtigt.

Die Beatmungshübe des Beatmungsgeräts 9 sowie die eigene Atmungsaktivität tragen dazu bei, das respiratorische System zu dehnen. Der gesamte vom Beatmungsgerät 9 erzeugte Druck bewirkt sowohl eine Dehnung der Lunge als auch eine Dehnung des Brustkorbs. Die Ummantelung der Lunge, genannt das Lungenfell, gleitet beim Atmen entlang der Innenseite des Brustkorbs. Zwischen Lunge und Brustkorb befindet sich der dünne Pleuraspalt. Dieser Pleuraspalt ist mit Flüssigkeit gefüllt und hält durch Kapillarkräfte die beiden aneinander angrenzenden Oberflächen der Lunge und des Brustkorbs zusammen.

Ein gutes Maß für den pneumatischen Druck, der bei einer künstlichen Beatmung auf die Lunge einwirkt, und somit ein Maß für den transpulmonalen Druck ist die Differenz aus dem gesamten Druck, der am respiratorischen System des Patienten P anliegt, und dem Druck im Pleuraspalt. Als Maß für den gesamten anliegenden Druck wird der Druck am Atemweg, bevorzugt der vom Sensor 3 gemessene airway pressure $P_{aw}$, verwendet. Der Druck am Pleuraspalt tritt zwischen der Außenseite der Lunge (Lungenfell) und der Innenseite (Rippenfell) des Brustkorbs (chestwall) auf. Diese Druck-Differenz berücksichtigt quantitativ sowohl die künstliche Beatmung durch das Beatmungsgerät 9 als auch die eigene Atmungsaktivität des Patienten P. Indem diese Differenz berücksichtigt wird, wird in dem gesamten Druck, der auf das respiratorische System einwirkt, der auf die Lunge einwirkende Druck von dem auf den Brustkorb einwirkenden Druck rechnerisch getrennt und dadurch der auf die Lunge einwirkende Druck isoliert ermittelt.

Der Pleuraspalt stellt ein geschlossenes System dar. Daher ist es nicht möglich, diesen pleuralen Druck direkt zu messen. Eine gute Näherung für den pleuralen Druck ist der Druck $P_{es}$, den die Sonde 10 in der Speiseröhre Sp des Patienten P misst - vorausgesetzt die Sonde 10 ist korrekt in der Speiseröhre Sp positioniert.

Um zu vermeiden, dass die Lunge des Patienten P in sich zusammenfällt und / oder zu stark gedehnt wird, wird ein Maß für die mechanische Dehnbarkeit oder Elastizität der Lunge gemessen. Diese Dehnbarkeit wird auch als Compliance der Lunge bezeichnet. Die Dehnbarkeit ist der Quotient aus bewirkter Änderung $\Delta$Vol (Vergrößerung oder Verkleinerung) des Lungenvolumens Vol und Änderung $\Delta$P des an der Lunge anliegenden Drucks, der diese Volumenänderung bewirkt, also Compliance = $\Delta$Vol / $\Delta$P.

Im Ausführungsbeispiel wird als Druckdifferenz, der auf die Lunge einwirkt und eine Volumenänderung bewirkt, eine Veränderung des transpulmonalen Drucks $P_{tp}$ verwendet. Als Maß für die Dehnbarkeit (Elastizität) der Lunge wird also der Quotient $\Delta$Vol / $\Delta P_{tp}$ verwendet.

Der transpulmonale Druck $P_{tp}$ ist ein Maß für die mechanische Belastung, welcher die Lunge aufgrund des Ausdehnens und Zusammenziehens beim Atmen ausgesetzt ist. Bevorzugt bezieht der transpulmonale Druck $P_{tp}$ sich auf einen Referenzwert, beispielsweise auf den Umgebungsluftdruck, und kann daher negative Werte annehmen. Der endinspiratorische transpulmonale Druck EIPtp ist ein Maß für die beim Atmen und bei der künstlichen Beatmung auftretende maximale Dehnung der Lunge, die nicht zu groß werden darf. Der endexspiratorische transpulmonale Druck EEPtp ist ein Maß für die minimale Dehnung und kann die Gefahr anzeigen, dass die Lunge kollabiert, insbesondere bei negativen Werten der Dehnung relativ zum Umgebungsdruck.

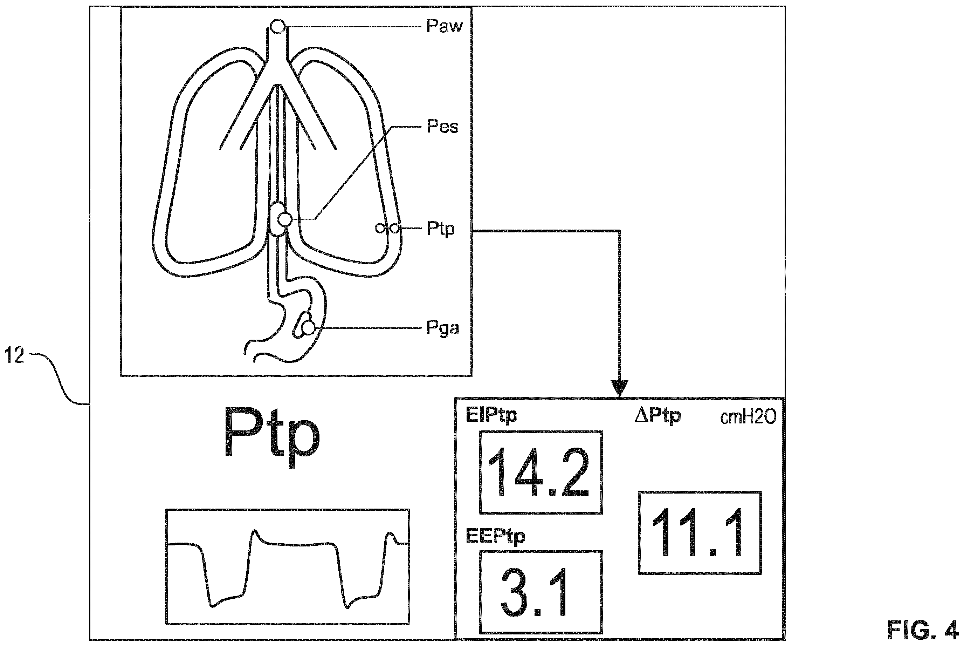

In einer Ausgestaltung werden die aktuellen Werte dieser drei Parameter auf der Anzeigeeinheit 12 dargestellt. Figur 4 zeigt beispielhaft, wie die aktuellen Werte der drei Parameter Ptp, EIPtp und EEPtp sowie der Wert $\Delta P_{tp}$ auf der Anzeigeeinheit 12 dargestellt werden. Außerdem zeigt Figur 4 den zeitlichen Verlauf des transpulmonalen Drucks $P_{tp}$.

Der transpulmonale Druck $P_{tp}$ lässt sich nicht direkt messen. In einer Ausgestaltung wird der transpulmonale Druck $P_{tp}$ als die Differenz aus dem Druck $P_{aw}$ am Atemweg und dem Druck $P_{es}$ in der Speiseröhre Sp berechnet, also

$$P_{tp} = P_{aw} - P_{es}.$$

Entsprechend gilt für die in Figur 2 bis Figur 4 dargestellten Parameter:

$$EIPtp = EIP - EIPes$$

und

$$EEPtp = PEEP - EEPes.$$

Eine Alternative, um den transpulmonalen Druck $P_{tp}$ zu ermitteln, benötigt nicht den Druck in der Speiseröhre Sp und kommt daher ohne eine Sonde 10 aus. Ein solches Verfahren wird in EP 2 397 074 B1 beschrieben. Bei dieser Alternative wird die Differenz $\Delta$Ptp zwischen dem endinspiratorischen transpulmonalen Druck EIPtp und dem endexspiratorischen transpulmonalen Druck EEPtp ermittelt. Das Beatmungsgerät 9 führt die künstliche Beatmung so durch, dass nacheinander zwei unterschiedliche Werte peep1 und peep2 für den endexspiratorischen Druck PEEP erzeugt werden, wobei peep2 der größere Wert ist. Jeder Wert führt zu jeweils einen Wert eelv1, eelv2 für das endexspiratorische Lungenvolumen EELV. Die Druckänderung peep2 - peep1 bewirkt eine Volumenänderung eelv2 - eelv1. Die gesuchte Differenz $\Delta$Ptp wird beispielsweise gemäß der Rechenvorschrift

$$\Delta Ptp = (eelv2 - eelv1) / (peep2 - peep1)$$

berechnet.

Die Dehnbarkeit (Compliance) des respiratorischen Systems umfassend Lunge und Brustkorb wird im Folgenden mit $C_{resp}$ bezeichnet, die Dehnbarkeit der Lunge mit $C_{Lung}$ und die Dehnbarkeit der Brustwand mit $C_{cw}$. Der Kehrwert 1/C wird auch als Elastance E bezeichnet. Es gilt:

$$1/C_{resp} = 1/C_{Lung} + 1/C_{cw} \text{ sowie } E_{resp} = E_{Lung} + EC_{cw}.$$

Bekanntlich ist die Dehnbarkeit eines Systems der Quotient aus

- der bewirkten Volumenänderung und
- dem Druck, der diese Volumenänderung bewirkt.

Das respiratorische System des Patienten P wird beim Einatmen durch den anliegenden Druck gedehnt und zieht sich beim Ausatmen wieder zusammen. Als Volumendifferenz ΔVol lässt sich das Tidalvolumen VT verwenden. Die durchschnittliche Dehnbarkeit des respiratorischen Systems lässt sich näherungsweise mithilfe der folgenden lungenmechanischen Modellgleichungen beschreiben:

$$C_{resp} = VT / \Delta P_{aw},$$

$$C_{Lung} = VT / \Delta P_{tp},$$

$$C_{Cw} = VT / \Delta P_{es}.$$

In einer Ausgestaltung misst der Sensor 3 vor dem Mund des Patienten P zusätzlich zu dem Atemwegsdruck $P_{aw}$ den Volumenstrom Vol' in den und aus dem Atemweg des Patienten P, also die Menge pro Zeiteinheit des bewegten Gases. In einer anderen Ausgestaltung misst der optionale Flusssensor 15 am Beatmungsgerät 9 diesen Volumenstrom Vol'. Aus diesem Volumenstrom Vol' lässt sich eine Volumenänderung und insbesondere das Tidalvolumen VT, also das Volumen, das die Lunge bei einem einzelnen Einatmungsvorgang aufnimmt, herleiten. Die Lunge vergrößert sich um dieses Volumen.

Möglich ist, die Dehnbarkeit der Lunge mithilfe dieses Volumenstroms Vol' und dem transpulmonalen Druck $P_{tp}$ herzuleiten. Dies liefert eine durchschnittliche Dehnbarkeit. Jedoch variiert in der Regel die Dehnbarkeit der Lunge von Bereich zu Bereich der Lunge. Einzelne Bereiche der Lunge können deutlich weniger dehnbar sein als andere Bereiche. Daher wird dieses globale Verfahren in der Regel nicht zu befriedigenden Ergebnissen führen. Um die lokale Dehnbarkeit der Lunge, also die Dehnbarkeit in bestimmten Bereichen, zu messen, wird im Ausführungsbeispiel die EIT-Messvorrichtung mit dem EIT-Gürtel 7 verwendet.

Die Grundidee eine solchen Messvorrichtung ist die folgende: Auf der Haut des Patienten P wird eine Abfolge von mindestens vier, beispielsweise 16 Elektroden gelegt. Zwischen zwei Elektroden dieser Abfolge wird ein Wechselstrom (Speisestrom) mit vorgegebener oder durch Messung bekannter Stromstärke eingespeist. Diese beiden Elektroden werden auch als Stimulations-Elektrodenpaar bezeichnet. An die übrigen Elektroden wird die Spannung, die aus der Einspeisung resultiert, gemessen. Die Impedanz Z des Gewebes zwischen den beiden Elektroden des Stimulations-Elektrodenpaars wird gemäß dem Ohmschen Gesetz als Quotient aus den gemessenen Werten für die Spannung an den übrigen Elektroden und der bekannten Einspeise-Stromstärke hergeleitet. Muskulatur und Blut im Körper des Patienten P können den eingespeisten Messstrom besser leiten als das Lungengewebe, weil Muskulatur und Blut mehr ungebundene Ionen aufweisen. Welche beiden Elektroden als Stimulations-Elektrodenpaar verwendet werden, wird im Laufe der Zeit mit hoher Frequenz automatisch verändert, wobei jede Elektrode in jeweils einem Teil des Mess-Zeitraums zu dem Stimulations-Elektrodenpaar gehört.

Je höher der Gehalt an Luft in einen Bereich der Lunge ist, desto größer ist die gemessene Impedanz in diesem Bereich. In der Regel vergrößert sich bei der Einatmung (durch eigene Atmungsaktivität und / oder künstliche Beatmung erzeugt) der Luftgehalt und damit die Impedanz in einem Bereich und verkleinert sich wieder bei der Ausatmung. Die regionale Differenz zwischen der Impedanz am Ende der Einatmung und der Impedanz am Ende der Ausatmung korreliert mit der regionalen Änderung des Luftgehalts in der Lunge. Als diese Korrelation kann ein linearer Zusammenhang verwendet werden, der z.B. vorab bei einer Kalibrierung ermittelt wird. Daher liefert das EIT-Messverfahren ein Abbild der regionalen Änderungen des Luftgehalts in der Lunge im Körper.

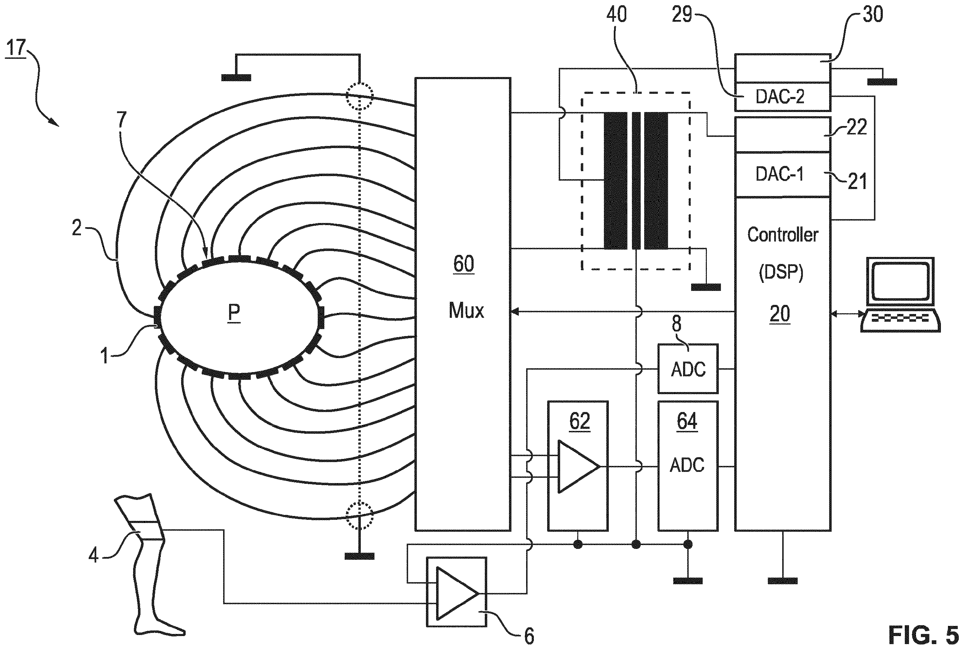

Figur 5 zeigt beispielhaft eine EIT-Messvorrichtung 17, wie sie z.B. in DE 10 2005 031 752 B4 beschrieben wird. EIT steht für elektrische Impedanztomografie. Um den Körper des Patienten P ist ein EIT-Gürtel 7 gelegt, welcher zu der EIT-Messvorrichtung 17 gehört. Dieser EIT-Gürtel 7 umfasst eine Vielzahl von Messelektroden 1, im gezeigten Beispiel 16 Messelektroden 1.

Jede Messelektrode 1 ist über jeweils ein Kabel 2 mit einem Umschalter oder Multiplexer 60 verbunden. Der Umschalter oder Multiplexer 60 legt ein Wechselstromsignal an jeweils zwei Messelektroden 1 an, die dann als Stimulations-Elektrodenpaar fungieren. Die Auswahl des Stimulations-Elektrodenpaars läuft um den Körper des Patienten P herum. Die übrigen 14 Messelektroden 1 fungieren als Messelektroden. Die Spannungssignale der Messelektroden 1 werden über den Umschalter oder Multiplexer 60, einen Differenzverstärker 62 und einen Analog-Digital-Wandler 64 einer Steuer- und Auswerteeinheit 20 zugeführt. Die Steuer- und Auswerteeinheit 20 erzeugt aus den Spannungssignalen ein Abbild der regionalen Verteilung des Luftgehalts in der Lunge. Um dieses Abbild zu erzeugen, wendet die Steuer- und Auswerteeinheit 20 ein geeignetes Verfahren zur Bildrekonstruktion an. Das erzeugte Abbild lässt sich als ein Elektroimpedanztomographie-Bild (EIT-Bild) bezeichnen.

Die beiden jeweils aktivierten Messelektroden 1 werden von einer Wechselstromquelle 22 mit Wechselstrom versorgt. Die Steuer- und Auswerteeinheit 20 ist über einen Digital-Analog-Wandler 21 mit der Wechselstromquelle 22 verbunden und steuert diese an. Der Wechselstrom der Wechselstromquelle 22 ist mittels eines Trenntransformators oder Übertragers 40 galvanisch von dem Umschalter oder Multiplexer 60 getrennt.

Beispielsweise am rechten Bein des Patienten P ist eine Messelektrode 4 befestigt, welche das Gleichtaktsignal am Körper des Patienten P gegenüber Masse misst. Die Signale der Messelektrode 4 werden über einen Messverstärker 6 und einen Analog-Digital-Wandler 8 der Steuer- und Auswerteeinheit 20 zugeführt. Über einen Digital-Analog-Wandler 29 ist die Steuer- und Auswerteeinheit 20 mit einer Kompensations-Wechselstromquelle 30 verbunden. Die Steuer- und Auswerteeinheit 20 steuert die Kompensations-Wechselstromquelle 30 nach Phase und Amplitude so an, dass die Symmetrie der primären Wechselstromquelle 22 an der Sekundärseite des Trenntransformators 40 verstimmt wird, und zwar so, dass das Gleichtaktsignal am Körper des Patienten P minimiert wird. Für diese Regelung verwendet die Steuer- und Auswerteeinheit 20 das Gleichtaktsignal, welches von der Messelektrode 4 gemessen und anschließend verstärkt wird.

Mithilfe eines derartigen EIT-Messvorrichtung 17 wird die regionale Änderung der Impedanz und damit die regionale Änderung des Luftvorrats in der Lunge des Patienten P ermittelt. Gesucht ist die regionale Dehnbarkeit (Compliance) $C_{Lung}[Reg]$, wobei die regionale Dehnbarkeit sich auf einen Bereich Reg der Lunge des Patienten P bezieht. Die Berechnung der jeweiligen Dehnbarkeit soll für mehrere Bereiche oder sogar für jeden Bereich der Lunge durchgeführt werden. Der oder jeder Bereich Reg ist so gewählt, dass die Dehnbarkeit in diesem Bereich zu einem Zeitpunkt als für den gesamten Bereich gleich angesehen werden kann. Mit $C_{resp}[Reg]$ wird die regionale Dehnbarkeit des respiratorischen Systems des Patienten P bezeichnet. Die regionale Dehnbarkeit eines Lungenbereichs kann mit der Zeit variieren. In einer Ausgestaltung ist jeder Bildpunkt (Pixel) des erzeugten EIT-Bilds der Lunge jeweils ein Bereich Reg. Auch größere Bereiche sind möglich, also Bereiche mit mehreren Bildpunkten des EIT-Bildes. Wie bereits dargelegt, hängt die Dehnbarkeit von der Druckänderung und der bewirken Volumenänderung ab. Die bewirkte Volumenänderung wird so wie gerade beschrieben von der EIT-Messvorrichtung 17 ermittelt.

Im Ausführungsbeispiel werden folgende lungenmechanischen Modellgleichungen verwendet:

$$C_{resp}[Reg] = \Delta Z[Reg] / \Delta P_{aw},$$

$$C_{Lung}[Reg] = \Delta Z[Reg] / \Delta P_{tp},$$

$$C_{CW}[Reg] = \Delta Z[Reg] / \Delta P_{es}.$$

Hierbei bezeichnet $\Delta Z[Reg]$ die Änderung des elektrischen Widerstands (Impedanz) in dem Bereich Reg, also die Änderung bezogen auf einen vorgegebenen Referenzwert. Diese regionale Änderung der Impedanz wird so wie gerade beschrieben von der EIT-Messvorrichtung ermittelt.

Um die regionale Dehnbarkeit $C_{Lung}[Reg]$ des Bereichs Reg der Lunge zu ermitteln, werden Messungen bei unterschiedlichen Einstellungen des Beatmungsgeräts 9 vorgenommen. In einer Ausgestaltung führt das Beatmungsgerät 9 automatisch ein Manöver aus. Möglich ist, dass eine zeitlich veränderliche Führungsgröße für dieses Manöver von einer Person, die das Manöver überwacht, vorgegeben wird. Bei diesem Manöver wird der endexspiratorische Druck PEEP zunächst auf einen relativ hohen Wert eingestellt, z.B. auf den größtmöglichen Wert, bei welchem das respiratorische System des Patienten P mit Sicherheit nicht beschädigt wird. Dadurch wird das respiratorische System des Patienten P geöffnet. Anschließend wird der endexspiratorische Druck PEEP schrittweise, also inkrementell, auf einen kleineren Wert als zuvor eingestellt. Auch beim kleinsten Wert ist die Gefahr ausreichend gering, dass die Lunge kollabiert. Bei jedem eingestellten Wert für den endexspiratorischen Druck PEEP wird jeweils ein Ermittlungsvorgang durchgeführt. Beispielsweise wird der endexspiratorische Druck PEEP nacheinander auf die elf Werte 25 cm H2O, 23 cm H2O, ..., 5 cm H2O eingestellt.

Selbstverständlich ist es umgekehrt möglich, zunächst mit einem kleineren Wert zu beginnen und dann den Wert schrittweise zu vergrößern. Bei dieser Alternative wird aber nicht das respiratorische System des Patienten P zu Beginn des Manövers geöffnet.

Jeder Ermittlungsvorgang bei einem eingestellten Wert für den endexspiratorischen Druck PEEP umfasst die folgenden Schritte, welche das Steuergerät 16 automatisch durchführt:

- Wiederholt werden der Atemwegsdruck $P_{aw}$ sowie der Druck $P_{es}$ in der Speiseröhre Sp gemessen. Bevorzugt ist die Abtast-Frequenz so groß, dass die beiden Drücke $P_{aw}$ sowie $P_{es}$ in der Zeitspanne zwischen dem Ende eines Einatmungsvorgangs und dem Beginn des nachfolgenden Ausatmungsvorgangs sowie in der Zeitspanne zwischen dem Ende eines Ausatmungsvorgangs und dem Beginn des nachfolgenden Einatmungsvorgangs jeweils mindestens einmal gemessen werden. Eine Messung während dieser beiden Zeitspannen ist in der Regel zuverlässiger als während einer anderen Zeitspanne, weil in diesen beiden Zeitspannen nur relativ wenig, idealerweise überhaupt keine, Luft in die Lunge oder aus der Lunge des Patienten P fließt, sodass die beiden Drücke in diesen beiden Zeitspannen annähernd konstant bleiben.

- Der transpulmonale Druck wird in einer Ausgestaltung gemäß der Rechenvorschrift $P_{tp} = P_{aw} - P_{es}$ hergeleitet, also unter Verwendung von Messwerten der Sensoren 3 und 10. Gemäß dieser Rechenvorschrift werden Werte für den endinspiratorischen transpulmonalen Druck EIPtp und für den endexspiratorischen transpulmonalen Druck EEPtp hergeleitet.

- Als Maß für den Druck, der diese Volumenänderung der Lunge bewirkt, wird die Differenz ΔPtp = EIPtp - EEPtp hergeleitet. Eine andere Darstellung dieser Herleitung ist die folgende Rechenvorschrift: ΔPtp = (EIP - EIPes) - (PEEP - EEPes).

- In einer anderen Ausgestaltung wird die Differenz ΔPtp gemäß der Rechenvorschrift ΔPtp = (eelv2 - eelv1) / (peep2 - peep1) berechnet. Hierbei ist peep1 der aktuelle Wert und peep2 der vorige Wert für PEEP.

- Außerdem wird die regionale Änderung ΔZ[Reg] des elektrischen Widerstands hergeleitet, wofür Messwerte von der EIT-Messvorrichtung 17 verwendet werden.

- Die regionale Dehnbarkeit $C_{Lung}$[Reg] der Lunge in einem Bereich Reg wird gemäß der Rechenvorschrift $C_{Lung}$[Reg] = ΔZ[Reg] / $ΔP_{tP}$ hergeleitet.

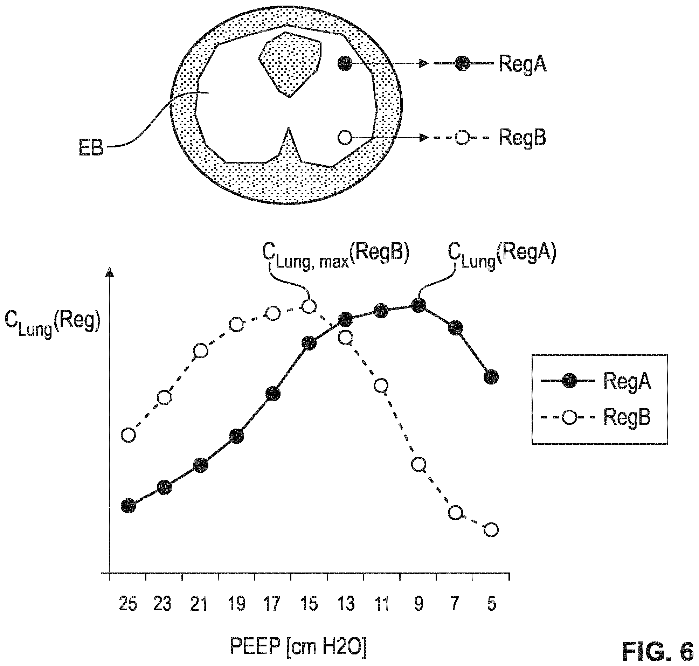

Figur 6 oben veranschaulicht zwei beispielhafte Bereiche RegA und RegB der Lunge des Patienten P. Üblicherweise liegt der Patient P bei einer künstlichen Beatmung auf dem Rücken, und das Eigengewicht des Patienten P wirkt dank der Schwerkraft auf den Körper des Patienten P ein, wobei der Körper sich senkrecht zur Schwerkraft ausdehnt. Der Bereich RegA liegt näher am Herzen, der Bereich RegB näher am Rücken des Patienten P. Bei einem liegenden Patienten P wirkt das Eigengewicht des Körpers deutlich stärker auf den Bereich RegB als auf den Bereich RegA ein. Diese beiden beispielhaften Bereiche RegA, RegB sind in dem EIT-Bild EB der Lunge sichtbar.

Figur 6 unten zeigt ein Diagramm. Auf der x-Achse ist der jeweils eingestellte Wert des endexspiratorischen Drucks PEEP in cm H2O dargestellt, auf der y-Achse die regionale Dehnbarkeit $C_{Lung}$[Reg], die so wie gerade geschrieben hergeleitet wurde. Gezeigt werden zwei Kurven $C_{Lung}$[RegA] und $C_{Lung}$[RegB].

Zu sehen ist, dass die regionale Dehnbarkeit $C_{Lung}$[RegA], $C_{Lung}$[RegB] als Funktion des eingestellten endexspiratorischen Drucks PEEP ein Maximum $C_{Lung,max}$[RegA], $C_{Lung,max}$[RegB] annimmt. Dieses Maximum variiert in der Regel von Bereich zu Bereich der Lunge. Ein wesentlicher Grund hierfür ist, dass das Eigengewicht des Körpers des Patienten P unterschiedlich stark auf diese Bereiche einwirkt. Andere beeinflussende Faktoren können die regionale Inflammation, inaktives Surfactant oder Ödeme sein. Im Folgenden wird mit $peep_{max}$[Reg] derjenige Wert des endexspiratorischen Drucks PEEP bezeichnet, bei dem die regionale Dehnbarkeit $C_{Lung}$[Reg] für diese Region Reg das Maximum annimmt. In der Regel variiert das Maximum von Bereich zu Bereich.

In einer Ausgestaltung wird für jeden Bereich Reg der Lunge und für jeden eingestellten Wert peep des endexspiratorischen Drucks PEEP jeweils ein Wert für die relative Steifigkeit $S_{Lung,rel}$[Reg] bezogen auf das Maximum berechnet, beispielsweise gemäß der Formel

$$S_{Lung,rel}[Reg](peep) =$$

$$\{C_{Lung,max}[Reg] - C_{Lung,max}[Reg](peep)\} / C_{Lung,max}[Reg] * 100\% =$$

$$\{1 - E_{Lung,max}[Reg] / E_{Lung,max}[Reg](peep)\} * 100\% .$$

$C_{Lung,max}$[Reg](peep) ist die regionale Compliance und $E_{Lung,max}$[Reg](peep) die regionale Elastance beim Wert peep für PEEP.

Diese relative Steifigkeit ist umso größer, je kleiner die regionale Dehnbarkeit ist.

In einer Ausgestaltung der künstlichen Beatmung soll in erster Linie verhindert werden, dass die Lunge des Patienten P kollabiert. Bei dieser Anwendung wird ein regionaler Kollabierungswert $K_{Lung,rel}$[Reg] gemäß der Rechenvorschrift

$$K_{Lung,rel}[Reg](peep) = S_{Lung,rel}[Reg](peep), \text{ falls } peep >= peep_{max}[Reg]$$

und

$$K_{Lung,rel}[Reg](peep) = 0, \text{ falls } peep < peep_{max}[Reg]$$

berechnet. Ein kumulierter Kollabierungswert $K_{Lung,rel}(peep)$ wird gemäß der Rechenvorschrift

$$K_{Lung,rel}(peep) = \Sigma \{ K_{Lung,rel}[Reg](peep) * C_{Lung,max}[Reg] \} / \Sigma C_{Lung,max}[Reg]$$

berechnet. Hierbei wird über alle Bereiche Reg der Lunge summiert. Dieser kumulierte Kollabierungswert hängt von dem Wert des endexspiratorischen Drucks PEEP ab. Das Beatmungsgerät 9 wird auf denjenigen Wert peep für den Druck PEEP eingestellt, der zu dem größten kumulierten Kollabierungswert $K_{Lung,rel}(peep)$ führt.

In einer anderen Ausgestaltung soll in erster Linie eine Überdehnung der Lunge verhindert werden. Bei dieser anderen Anwendung wird ein regionaler Überdehnungswert gemäß der Rechenvorschrift

$$Ü_{Lung,rel}[Reg](peep) = S_{Lung,rel}[Reg](peep), \text{ falls } peep < peep_{max}[Reg]$$

und

$$Ü_{Lung,rel}[Reg](peep) = 0, \text{ falls } peep >= peep_{max}[Reg]$$

berechnet. Ein kumulierter Überdehnungswert $Ü_{Lung,rel}(peep)$ wird gemäß der Rechenvorschrift

$$Ü_{Lung,rel}(peep) = \Sigma \{ Ü_{Lung,rel}[Reg](peep) * C_{Lung,max}[Reg] \} / \Sigma C_{Lung,max}[Reg]$$

berechnet, wobei wiederum über alle berücksichtigten Lungenbereiche summiert wird. Wiederum wird das Beatmungsgerät 9 auf denjenigen Wert eingestellt, der zu dem größten kumulierten Überdehnungswert $Ü_{Lung,rel}(peep)$ führt.

Diese beiden Ausgestaltungen lassen sich miteinander kombinieren.

### Bezugszeichenliste

| | |
|---|---|
| 1 | 16 Messelektroden des EIT-Gürtels 7 |
| 2 | Kabel, welche die Messelektroden 1 mit dem Multiplexer 60 verbinden |
| 3 | pneumatischer Sensor vor dem Mund des Patienten P, misst den Atemwegsdruck $P_{aw}$ und optional den Volumenstrom Vol', fungiert als ein Atemwegsdruck-Sensor |
| 3.1 | Messwertaufnehmer des Sensors 3, zweigt Luft aus dem Beatmungskreislauf ab |
| 3.2 | eigentlicher Drucksensor des Sensors 3, empfängt Fluidströme vom Messwertaufnehmer 3.1 |
| 4 | Messelektrode am Bein des Patienten P, misst ein Gleichtaktsignal des Körpers des Patienten P gegenüber Masse |
| 5.1.1, 5.1.2 | herznahes Paar von Messelektroden auf der Haut des Patienten P |
| 5.2.1, 5.2.2 | zwerchfellnahes Paar von Messelektroden auf der Haut des Patienten P |
| 6 | Messverstärker |
| 7 | EIT-Gürtel, um den Körper des Patienten P gelegt, umfasst eine Vielzahl (z.B. 16) von Messelektroden 1 und jeweils ein Kabel 2 pro Messelektrode 1, gehört zu der EIT-Messvorrichtung 17 |
| 8 | Analog-Digital-Wandler |

(fortgesetzt)

| | |
|---|---|
| 9 | Beatmungsgerät, beatmet den Patienten P künstlich, umfasst die Anzeigeeinheit 12 |
| 10 | Sonde in der Speiseröhre Sp des Patienten P, misst den Speiseröhren-Druck $P_{es}$ und optional den gastralen Druck $P_{ga}$ |
| 11 | Anschlussstück im Mund des Patienten P, mit dem Messkatheter 14 in der Speiseröhre Sp verbunden |
| 12 | Anzeigeeinheit des Beatmungsgeräts 9, umfasst einen Bildschirm |
| 13 | gastrale Sonde im Magen Ma des Patent P, misst den gastralen Druck $P_{ga}$ |
| 14 | Messkatheter in der Speiseröhre Sp des Patienten P, mit dem Anschlussstück 11 verbunden |
| 15 | Sensor am Beatmungsgerät 9, misst den Volumenstrom Vol' |
| 16 | Steuergerät, empfängt Signale von der EIT-Messvorrichtung 17 und von den Sensoren 3, 10, 15 |
| 17 | EIT-Messvorrichtung, umfasst den EIT-Gürtel 7 - EIT bedeutet elektrische Impedanztomografie |
| 20 | Steuer- und Auswerteeinheit der EIT-Messvorrichtung 17 |
| 21 | Digital-Analog-Wandler |
| 29 | Digital-Analog-Wandler |
| 30 | Kompensations-Wechselstromquelle |
| 60 | Multiplexer, mit dem die Kabel 2 verbunden sind |
| $C_{cw}$ | durchschnittliche Dehnbarkeit (Compliance) des Brustkorbs (chestwall) des Patienten P |
| CLung | durchschnittliche Dehnbarkeit (Compliance) der Lunge des Patienten P |
| $C_{Lung}[Reg]$ | regionale Dehnbarkeit (Compliance) der Lunge im Bereich Reg |
| $C_{Lung}[Reg](peep)$ | Wert für den Wert peep der regionalen Dehnbarkeit (Compliance) der Lunge im Bereich Reg |
| Chung,max[Reg] | maximale regionale Dehnbarkeit (Compliance) der Lunge im Bereich Reg |
| $C_{resp}$ | durchschnittliche Dehnbarkeit (Compliance) des respiratorischen Systems bestehend aus Lunge und Brustwall des Patienten P |
| Cresp[Reg] | regionale Dehnbarkeit (Compliance) des respiratorischen Systems in einem Bereich Reg |
| $\Delta P$ | Änderung des an der Lunge anliegenden Drucks |
| $\Delta Paw$ | Differenz zwischen dem endinspiratorischen Druck EIP und dem endexspiratorischen Druck PEEP |
| $\Delta Pes$ | Differenz zwischen dem endinspiratorischen Druck EIPes und dem endexspiratorischen Druck EEPes |
| $\Delta Ptp$ | Differenz zwischen dem endinspiratorischen transpulmonalen Druck EIPtp und dem endexspiratorischen transpulmonalen Druck EEPtp |
| $\Delta Vol$ | Änderung des Lungenvolumens, die durch die Änderung $\Delta P$ des an der Lunge anliegenden Drucks bewirkt wird |
| $\Delta Z[Reg]$ | Änderung des elektrischen Widerstands (Impedanz) in dem Lungenbereich Reg |
| EEPes | endexspiratorischer Druck in der Speiseröhre Sp, also Druck $P_{es}$ am Ende eines Ausatmungsvorgangs |
| EIP | endinspiratorischer Druck am Atemweg des Patienten P, also Druck $P_{aw}$ am Ende eines Einatmungsvorgangs |
| EIPes | endinspiratorischer Druck in der Speiseröhre Sp, also Druck $P_{es}$ am Ende eines Einatmungsvorgangs |

(fortgesetzt)

| | |
|---|---|
| EB | EIT-Bild der Lunge des Patienten P |
| EEPtp | endexspiratorischer transpulmonaler Druck, also Druck $P_{tp}$ am Ende eines Ausatmungsvorgangs |
| EIPtp | endinspiratorischer transpulmonaler Druck, also transpulmonaler Druck $P_{tp}$ am Ende eines Einatmungsvorgangs |
| KLung,rel[Reg] | regionaler Kollabierungswert im Bereich Reg |
| Lu | Lunge des Patienten P |
| Ma | Magen des Patienten P |
| P | Patient, der künstlich beatmet wird und spontan atmet, hat die Lunge Lu, den Magen Ma, die Speiseröhre Sp und das Zwerchfell Zw |
| $P_{aw}$ | pneumatischer Druck am Atemweg (airway pressure), wird mithilfe des Sensors 3 gemessen |
| $P_{di}$ | Druck am Zwerchfell, als Differenz $P_{gs}$ - $EEP_{es}$ ermittelt |
| $P_{es}$ | pneumatischer Druck in der Speiseröhre Sp des Patienten, mithilfe der Sonde 10 gemessen |
| $P_{ga}$ | gastraler Druck im Magen Ma des Patienten P, wird mithilfe der Sonde 10 oder einer gastralen Sonde gemessen |
| $P_{tp}$ | transpulmonaler Druck, bevorzugt als Differenz $P_{aw}$ - $P_{es}$ ermittelt |
| PEEP | endexspiratorischer Druck am Atemweg, also Druck $P_{aw}$ am Ende eines Ausatmungsvorgangs, lässt sich am Beatmungsgerät 9 einstellen |
| peep | Wert für PEEP |
| $peep_{max}$[Reg] | derjenige Wert für PEEP, bei dem die regionale Dehnbarkeit $C_{Lung}$[Reg] für die Region Reg das Maximum annimmt |
| RegA, RegB | beispielhafte Bereiche der Lunge des Patienten P |
| $S_{Lung,rel}$[Reg](peep) | relative Steifigkeit des Bereichs Reg der Lunge beim Wert peep für PEEP |
| Sp | Speiseröhre des Patienten P, nimmt die Sonde 10 auf |
| Tb | Tidalbild von der Lunge des Patienten P, mithilfe der EIT-Messvorrichtung 17 erzeugt |
| Übung, rel[Reg] | regionaler Überdehnungswert im Bereich Reg |
| Vol' | Volumenfluss zwischen Patient P und Beatmungsgerät 9 |
| Zw | Zwerchfell des Patienten P |

**Patentansprüche**

1.  Anordnung zur automatischen Ermittlung eines Maßes für die jeweilige regionale Dehnbarkeit {$C_{Lung}$[RegA], $C_{Lung}$[RegB]} der Lunge eines Patienten (P) in mehreren unterschiedlichen vorgegebenen Bereichen (RegA, RegB) der Lunge,

    wobei die Anordnung

    - eine EIT-Messvorrichtung (17) zur Messung eines Maßes für die Volumenänderung eines Lungenbereichs (RegA, RegB),
    - einen pneumatischen Atemwegsdruck-Sensor (3),
    - einen pneumatischen Speiseröhrendruck-Sensor (10) und
    - ein datenverarbeitendes Steuergerät (16)

    umfasst,

wobei die EIT-Messvorrichtung (17) dazu ausgestaltet ist, ein Maß für die jeweilige Volumenänderung jedes Lungenbereichs (RegA, RegB) mittels Elektroimpedanztomographie (EIT) zu messen, wobei der Atemwegsdruck-Sensor (3) dazu ausgestaltet ist, ein Maß für den zeitlich veränderlichen Druck ($P_{aw}$) am Atemweg des Patienten (P) zu messen, wobei der Speiseröhrendruck-Sensor (10) dazu ausgestaltet ist, ein Maß für den zeitlich veränderlichen Druck ($P_{es}$) in der Speiseröhre (Sp) des Patienten (P) zu messen, und wobei das Steuergerät (16) dazu ausgestaltet ist,

- unter Verwendung von Signalen der EIT-Messvorrichtung (17) für jeden Bereich (RegA, RegB) der Lunge jeweils ein Maß für die Differenz zwischen dem endinspiratorischen und dem endexspiratorischen Volumen dieses Bereichs (RegA, RegB) der Lunge zu ermitteln, unter Verwendung von Signalen des Atemwegsdruck-Sensors (3) ein Maß für die Differenz ($\Delta$Ptp) zwischen dem an der Lunge anliegenden endinspiratorischen transpulmonalen Druck (EIPtp) und dem an der Lunge anliegenden endexspiratorischen transpulmonalen Druck (EEPtp) zu ermitteln und
- als Maß für die regionale Dehnbarkeit {$C_{Lung}$[RegA], $C_{Lung}$[RegB]} eines Bereichs (RegA, RegB) der Lunge

den Quotienten aus
der Volumen-Differenz für diesen Bereich (RegA, RegB) und
der an der Lunge anliegenden Druck-Differenz ($\Delta$Ptp) zu berechnen, und

wobei das Steuergerät (16) dazu ausgestaltet ist,

- den endinspiratorischen transpulmonalen Druck (EIPtp) als Differenz zwischen dem endinspiratorischen Atemwegsdruck (EIP) und dem endinspiratorischen Speiseröhren-Druck (EIPes) zu ermitteln und
- den endexspiratorischen transpulmonalen Druck (EEPtp) als Differenz zwischen dem endexspiratorischen Atemwegsdruck (PEEP) und dem endexspiratorischen Speiseröhren-Druck (EEPes) zu ermitteln.

2. Anordnung nach Anspruch 1,
**dadurch gekennzeichnet, dass**

die Anordnung wenigstens zeitweise in einer Datenverbindung mit einem Beatmungsgerät (9) steht, welches dazu ausgestaltet ist, den Patienten (P) künstlich zu beatmen, wobei das Beatmungsgerät (9) dazu ausgestaltet ist, den Patienten (P) dergestalt künstlich zu beatmen, dass der endexspiratorische Druck (PEEP) am Atemweg des Patienten (P) einen vorgegebenen Wert (peep) annimmt, und wobei das Steuergerät (16) dazu ausgestaltet ist, das Beatmungsgerät (9) dergestalt anzusteuern, dass das angesteuerte Beatmungsgerät (9) eine künstliche Beatmung dergestalt durchführt, dass der endexspiratorische Druck (PEEP) zunächst einen vorgegebenen ersten Wert (peep1) und anschließend mindestens einen vorgegebenen zweiten Wert (peep2), der vom ersten Wert (peep1) verschieden ist, annimmt.

3. Anordnung nach Anspruch 2,
**dadurch gekennzeichnet, dass**

das Steuergerät (16) dazu ausgestaltet ist,
für jeden vorgegebenen Bereich (RegA, RegB) der Lunge und
für den ersten Wert (peep1) und für den oder mindestens einen zweiten Wert (peep2), den der endexspiratorische Druck (PEEP) bei der künstlichen Beatmung annimmt,
die bei diesem Wert (peep1, peep2) jeweils resultierende regionale Dehnbarkeit der Lunge in diesem Lungenbereich (RegA, RegB) zu ermitteln.

4. Anordnung nach Anspruch 3,
**dadurch gekennzeichnet, dass**

das Steuergerät (16) dazu ausgestaltet ist, einen geforderten Betriebswert für den endexspiratorischen Druck (PEEP) am Atemweg zu berechnen,
wobei das Steuergerät (16) dazu ausgestaltet ist,
bei der Berechnung des geforderten Betriebswerts

den geforderten Betriebswert abhängig von einem Maß für die Dehnbarkeit der Lunge zu berechnen und das Maß für die Lungen-Dehnbarkeit durch Kumulieren über die regionalen Dehnbarkeiten {$C_{Lung}$[RegA], $C_{Lung}$[RegB]} der Lungenbereiche (RegA, RegB) für die mindestens zwei Werte des endexspiratorischen Drucks (PEEP) zu berechnen.

5. Anordnung nach einem der Ansprüche 2 bis 4,
**dadurch gekennzeichnet, dass**
die Anordnung dazu ausgestaltet ist,

- ein Maß für das jeweilige endexspiratorische Lungenvolumen beim ersten Wert (peep1) und beim zweiten Wert (peep2) des endexspiratorischen Drucks (PEEP) zu ermitteln und
- als das Maß für die Differenz zwischen dem endinspiratorischen transpulmonalen Druck (EIPtp) und dem endexspiratorischen transpulmonalen Druck (EEPtp)

den Quotienten aus
der Differenz zwischen den beiden gemessenen endexspiratorischen Lungenvolumina und
der Differenz zwischen den beiden eingestellten Werten (peep1, peep2) für den endexspiratorischen Druck (PEEP)
zu berechnen.

6. System zur künstlichen Beatmung eines Patienten (P),

wobei das System

- ein Beatmungsgerät (9) und
- eine Anordnung nach einem der vorhergehenden Ansprüche umfasst,

wobei das Beatmungsgerät (9) dazu ausgestaltet ist, eine künstliche Beatmung des Patienten (P) abhängig von den ermittelten regionalen Dehnbarkeiten {$C_{Lung}$[RegA], $C_{Lung}$[RegB]} der Lunge des Patienten (P) durchzuführen.

7. System nach Anspruch 6,
**dadurch gekennzeichnet, dass**

das Steuergerät (16) dazu ausgestaltet ist, das Beatmungsgerät (9) dergestalt anzusteuern, dass das angesteuerte Beatmungsgerät (9) eine künstliche Beatmung dergestalt durchführt, dass
der endexspiratorische Druck (PEEP) zunächst einen vorgegebenen ersten Wert (peep1) und anschließend mindestens einen vorgegebenen zweiten Wert (peep2), der vom ersten Wert (peep1) verschieden ist, annimmt, wobei das Steuergerät (16) dazu ausgestaltet ist,
für jeden vorgegebenen Bereich (RegA, RegB) der Lunge und
für den ersten Wert (peep1) und für den oder mindestens einen zweiten Wert (peep2), den der endexspiratorische Druck (PEEP) bei der künstlichen Beatmung annimmt,
die bei diesem Wert (peep1, peep2) jeweils resultierende regionale Dehnbarkeit der Lunge in diesem Lungenbereich (RegA, RegB) zu ermitteln.

8. Computerprogrammprodukt,

welches auf einem Steuergerät (16) ausführbar ist und
bei der Ausführung auf dem Steuergerät (16)
dann, wenn das Steuergerät (16)

- Signale von einer EIT-Messvorrichtung (17) zur Messung eines Maßes für die Volumenänderung eines Bereichs (RegA, RegB) der Lunge eines Patienten (P)
- Signale von einem pneumatischen Atemwegsdruck-Sensor (3) zur Messung eines Maßes für den zeitlich veränderlichen Druck ($P_{aw}$) am Atemweg des Patienten (P) und
- Signale von einem pneumatischen Speiseröhrendruck-Sensor (10) zur Messung eines Maßes für den zeitlich veränderlichen Druck ($P_{es}$) in der Speiseröhre (Sp) des Patienten (P)

empfängt,

bewirkt, dass das Steuergerät (16) ein Verfahren zur automatischen Ermittlung eines Maßes für die jeweilige regionale Dehnbarkeit {$C_{Lung}$[RegA], $C_{Lung}$[RegB]} der Lunge eines Patienten (P) in mehreren unterschiedlichen Bereichen (RegA, RegB) der Lunge durchführt,

wobei das Computerprogrammprodukt bewirkt, dass bei der Durchführung des Verfahrens die Schritte durchgeführt werden, dass

- die EIT-Messvorrichtung (17) mittels Elektroimpedanztomographie (EIT) ein Maß für die Volumen-Änderung des Lungenbereichs (RegA, RegB) misst,
- unter Verwendung von Signalen der EIT-Messvorrichtung (17) ein Maß für die Differenz zwischen dem endinspiratorischen und dem endexspiratorischen Volumen des Lungenbereichs ermittelt wird und
- als Maß für die regionale Dehnbarkeit {$C_{Lung}$[RegA], $C_{Lung}$[RegB]} des Lungenbereichs

der Quotienten aus der Volumen-Differenz für diesen Bereich und der an der Lunge anliegenden Druck-Differenz ($\Delta$Ptp)
berechnet wird,

wobei das Computerprogrammprodukt bewirkt, dass bei der Durchführung des Verfahrens die Schritte durchgeführt werden, dass

- der endinspiratorische transpulmonale Druck (EIPtp) als Differenz zwischen dem endinspiratorischen Atemwegsdruck (EIP) und dem endinspiratorischen Speiseröhren-Druck (EIPes) ermittelt wird und
- der endexspiratorische transpulmonale Druck (EEPtp) als Differenz zwischen dem endexspiratorischen Atemwegsdruck (PEEP) und dem endexspiratorischen Speiseröhren-Druck (EEPes) ermittelt wird.

## Claims

1. Arrangement for automatically determining a measure for the respective regional compliance {$C_{Lung}$[RegA], $C_{Lung}$[RegB]} of the lung of a patient (P) in a plurality of different specified regions (RegA, RegB) of the lung,

wherein the arrangement comprises

- an EIT measuring device (17) for measuring a measure for the change in volume of a lung region (RegA, RegB),
- a pneumatic airway pressure sensor (3),
- a pneumatic oesophageal pressure sensor (10) and
- a data-processing controller (16),

wherein the EIT measuring device (17) is designed to measure a measure for the respective change in volume of each lung region (RegA, RegB) by means of electrical impedance tomography (EIT),
wherein the airway pressure sensor (3) is designed to measure a measure for the time-varying pressure ($P_{aw}$) in the airway of the patient (P),
wherein the oesophageal pressure sensor (10) is designed to measure a measure for the time-varying pressure ($P_{es}$) in the oesophagus (Sp) of the patient (P) and
wherein the controller (16) is designed

- using signals of the EIT measuring device (17)

to determine, for each region (RegA, RegB) of the lung, a respective measure for the difference between the end-inspiratory and the end-expiratory volume of this region (RegA, RegB) of the lung,
using signals of the airway pressure sensor (3)
to determine a measure for the difference ($\Delta$Ptp) between the end-inspiratory transpulmonary pressure (EIPtp) present in the lung and the end-expiratory transpulmonary pressure (EEPtp) present in the lung and

- to calculate, as a measure for the regional compliance {$C_{Lung}$[RegA], $C_{Lung}$[RegB]} of a region (RegA, RegB) of the lung,

the quotient of
the volume difference for this region (RegA, RegB) and
the pressure difference ($\Delta$Ptp) present in the lung, and

wherein the controller (16) is designed

- to determine the end-inspiratory transpulmonary pressure (EIPtp) as difference between the end-inspiratory airway pressure (EIP) and the end-inspiratory oesophageal pressure (EIPes) and
- to determine the end-expiratory transpulmonary pressure (EEPtp) as difference between the end-expiratory airway pressure (PEEP) and the end-expiratory oesophageal pressure (EEPes).

2. Arrangement according to Claim 1,
**characterized in that**

the arrangement at least intermittently has a data connection to a ventilator (9), which is designed to artificially ventilate the patient (P),
wherein the ventilator (9) is designed to artificially ventilate the patient (P) in such a way that the end-expiratory pressure (PEEP) in the airway of the patient (P) assumes a specified value (peep), and
wherein the controller (16) is designed to control the ventilator (9) in such a way that the controlled ventilator (9) performs an artificial ventilation in such a way that
the end-expiratory pressure (PEEP) initially assumes a specified first value (peep1) and subsequently assumes at least one specified second value (peep2), which differs from the first value (peep1).

3. Arrangement according to Claim 2,
**characterized in that**

the controller (16) is designed,
for each specified region (RegA, RegB) of the lung and
for the first value (peep1) and for the second or at least one second value (peep2), which the end-expiratory pressure (PEEP) assumes during the artificial ventilation,
to determine the respective resultant regional compliance of the lung in this lung region (RegA, RegB) emerging for this value (peep1, peep2).

4. Arrangement according to Claim 3
**characterized in that**

the controller (16) is designed to calculate a required operational value for the end-expiratory pressure (PEEP) in the airway,
wherein the controller (16) is designed,
when calculating the required operational value,
to calculate the required operational value on the basis of a measure for the compliance of the lung and
to calculate the measure for the compliance of the lung by accumulating over the regional compliances $\{C_{Lung}[RegA], C_{Lung}[RegB]\}$ of the lung regions (RegA, RegB) for the at least two values of the end-expiratory pressure (PEEP).

5. Arrangement according to any one of Claims 2 to 4,
**characterized in that**
the arrangement is designed

- to determine a measure for the respective end-expiratory lung volume at the first value (peep1) and at the second value (peep2) of the end-expiratory pressure (PEEP) and
- to calculate, as the measure for the difference between the end-inspiratory transpulmonary pressure (EIPtp) and the end-expiratory transpulmonary pressure (EEPtp),

the quotient of
the difference between the two measured end-expiratory lung volumes and
the difference between the two set values (peep1, peep2) for the end-expiratory pressure (PEEP).

**6.** System for artificial ventilation of a patient (P),

wherein the system comprises

- a ventilator (9) and
- an arrangement according to any one of the preceding claims,

wherein the ventilator (9) is designed to perform artificial ventilation of the patient (P) on the basis of the determined regional compliances {$C_{Lung}$[RegA], $C_{Lung}$[RegB]} of the lung of the patient (P).

**7.** System according to Claim 6,
**characterized in that**

the controller (16) is designed to control the ventilator (9) in such a way that the controlled ventilator (9) performs an artificial ventilation in such a way that
the end-expiratory pressure (PEEP) initially assumes a specified first value (peep1) and subsequently assumes at least one specified second value (peep2), which differs from the first value (peep1),
wherein the controller (16) is designed,
for each specified region (RegA, RegB) of the lung and
for the first value (peep1) and for the second or at least one second value (peep2), which the end-expiratory pressure (PEEP) assumes during the artificial ventilation,
to determine the respective resultant regional compliance of the lung in this lung region (RegA, RegB) emerging for this value (peep1, peep2).

**8.** Computer program product,

which is executable on a controller (16) and,
when executed on the controller (16),
whenever the controller (16) receives

- signals from an EIT measuring device (17) for measuring a measure for the change in volume of a region (RegA, RegB) of the lung of a patient (P),
- signals from a pneumatic airway pressure sensor (3) for measuring a measure for the time-varying pressure ($P_{aw}$) in the airway of the patient (P) and
- signals from a pneumatic oesophageal pressure sensor (10) for measuring a measure for the time-varying pressure ($P_{es}$) in the oesophagus (Sp) of the patient (P),

causes the controller (16) to perform a method for automatically determining a measure for the respective regional compliance {$C_{Lung}$[RegA], $C_{Lung}$[RegB]} of the lung of a patient (P) in a plurality of different regions (RegA, RegB) of the lung,
wherein the computer program product, during the performance of the method, causes the implementation of the steps that

- the EIT measuring device (17) measures a measure for the change in volume of the lung region (RegA, RegB) by means of electrical impedance tomography (EIT),
- a measure for the difference between the end-inspiratory and the end-expiratory volume of the lung region is determined using signals of the EIT measuring device (17) and,
- as a measure for the regional compliance {$C_{Lung}$[RegA], $C_{Lung}$[RegB]} of the lung region,

the quotient of the volume difference for this region and the pressure difference ($\Delta Ptp$) present in the lung is calculated,

wherein the computer program product, during the performance of the method, causes the implementation of the steps that

- the end-inspiratory transpulmonary pressure (EIPtp) is determined as difference between the end-inspiratory airway pressure (EIP) and the end-inspiratory oesophageal pressure (EIPes) and
- the end-expiratory transpulmonary pressure (EEPtp) is determined as difference between the end-expiratory airway pressure (PEEP) and the end-expiratory oesophageal pressure (EEPes).

**Revendications**

1. Agencement permettant de déterminer automatiquement une mesure de la compliance régionale respective {$C_{Lung}$[RegA], $C_{Lung}$[RegB]} des poumons d'un patient (P) dans plusieurs zones prédéfinies différentes (RegA, RegB) des poumons,

   dans lequel l'agencement comprend

   - un dispositif de mesure EIT (17) pour mesurer la mesure de la variation de volume d'une zone pulmonaire (RegA, RegB),
   - un capteur pneumatique de pression des voies respiratoires (3),
   - un capteur pneumatique de pression de l'œsophage (10), et
   - un appareil de commande à traitement de données (16),

   dans lequel le dispositif de mesure EIT (17) est conçu pour mesurer une mesure de la variation de volume respective de chaque zone pulmonaire (RegA, RegB) au moyen de la tomographie par impédance électrique (EIT),
   dans lequel le capteur de pression des voies respiratoires (3) est conçu pour mesurer une mesure de la pression variable dans le temps ($P_{aw}$) au niveau des voies respiratoires du patient (P),
   dans lequel le capteur de pression de l'œsophage (10) est conçu pour mesurer une mesure de la pression variable dans le temps ($P_{es}$) dans l'oesophage (Sp) du patient (P), et
   l'appareil de commande (16) est conçu pour

   - déterminer, en utilisant des signaux du dispositif de mesure EIT (17) pour chaque zone (RegA, RegB) des poumons, respectivement une mesure de la différence entre le volume de fin d'inspiration et le volume de fin d'expiration de cette zone (RegA, RegB) des poumons,
   déterminer, en utilisant des signaux du capteur de pression des voies respiratoires (3), une mesure de la différence ($\Delta$Ptp) entre la pression transpulmonaire de fin d'inspiration (EIPtp) présente au niveau des poumons et la pression transpulmonaire de fin d'expiration (EEPtp) présente au niveau des poumons, et
   - calculer comme la mesure de la compliance régionale {$C_{Lung}$[RegA], $C_{Lung}$[RegB]} d'une zone (RegA, RegB) des poumons, le quotient de la différence de volume pour cette zone (RegA, RegB) et de la différence de pression présente au niveau des poumons ($\Delta$Ptp), et

   dans lequel l'appareil de commande (16) est conçu pour

   - déterminer la pression transpulmonaire de fin d'inspiration (EIPtp) comme la différence entre la pression des voies respiratoires de fin d'inspiration (EIP) et la pression de l'œsophage de fin d'inspiration (EIPes), et
   - déterminer la pression transpulmonaire de fin d'expiration (EEPtp) comme la différence entre la pression des voies respiratoires de fin d'expiration (PEEP) et la pression de l'œsophage de fin d'expiration (EEPes).

2. Agencement selon la revendication 1, **caractérisé en ce que**

   l'agencement est au moins temporairement en liaison de données avec un appareil respiratoire (9) qui est conçu pour pratiquer une respiration artificielle du patient (P),
   dans lequel l'appareil respiratoire (9) est conçu pour pratiquer la respiration artificielle du patient (P) de telle sorte que la pression de fin d'expiration (PEEP) au niveau des voies respiratoires du patient (P) adopte une valeur prédéfinie (peep), et
   dans lequel l'appareil de commande (16) est conçu pour piloter l'appareil respiratoire (9) de telle sorte que l'appareil respiratoire piloté (9) effectue une respiration artificielle de telle sorte que la pression de fin d'expiration (PEEP) adopte d'abord une première valeur prédéfinie (peep1) et ensuite au moins une deuxième valeur prédéfinie (peep2) qui est différente de la première valeur (peep1).

3. Agencement selon la revendication 2, **caractérisé en ce que** l'appareil de commande (16) est conçu pour déterminer pour chaque zone prédéfinie (RegA, RegB) des poumons et pour la première valeur (peep1) et pour la ou au moins une deuxième valeur (peep2) qu'adopte la pression de fin d'expiration (PEEP) lors de la respiration artificielle, la compliance régionale résultant respectivement à cette valeur (peep1, peep2) des poumons dans cette zone pulmonaire (RegA, RegB).

**4.** Agencement selon la revendication 3, **caractérisé en ce que**

l'appareil de commande (16) est conçu pour calculer une valeur de service demandée pour la pression de fin d'expiration (PEEP) au niveau des voies respiratoires,
dans lequel l'appareil de commande (16) est conçu
pour calculer lors du calcul de la valeur de service demandée la valeur de service demandée en fonction d'une mesure de la compliance des poumons, et
pour calculer la mesure de la compliance pulmonaire en cumulant les compliances régionales {$C_{Lung}$[RegA], $C_{Lung}$[RegB]} des zones pulmonaires (RegA, RegB) pour les au moins deux valeurs de la pression de fin d'expiration (PEEP).

**5.** Agencement selon l'une quelconque des revendications 2 à 4, **caractérisé en ce que** l'agencement est conçu pour

- déterminer une mesure du volume pulmonaire de fin d'expiration respectif à la première valeur (peep1) et à la deuxième valeur (peep2) de la pression de fin d'expiration (PEEP), et
- calculer comme la mesure de la différence entre la pression transpulmonaire de fin d'inspiration (EIPtp) et la pression transpulmonaire de fin d'expiration (EEPtp) le quotient de la différence entre les deux volumes pulmonaires de fin d'expiration mesurés et de la différence entre les deuxièmes valeurs réglées (peep1, peep2) pour la pression de fin d'expiration (PEEP).

**6.** Système pour pratiquer une respiration artificielle d'un patient (P),
dans lequel le système comprend

- un appareil respiratoire (9), et
- un agencement selon l'une quelconque des revendications précédentes,

dans lequel l'appareil respiratoire (9) est conçu pour effectuer une respiration artificielle du patient (P) en fonction des compliances régionales déterminés {$C_{Lung}$[RegA], $C_{Lung}$[RegB]} des poumons du patient (P).

**7.** Système selon la revendication 6, **caractérisé en ce que**

l'appareil de commande (16) est conçu piloter pour l'appareil respiratoire (9) de telle sorte que l'appareil respiratoire piloté (9) effectue une respiration artificielle de telle sorte que la pression de fin d'expiration (PEEP) adopte d'abord une première valeur prédéfinie (peep1) et ensuite au moins une deuxième valeur prédéfinie (peep2) qui est différente de la première valeur (peep1),
dans lequel l'appareil de commande (16) est conçu pour déterminer pour chaque zone prédéfinie (RegA, RegB) des poumons et pour la première valeur (peep1) et pour la ou au moins une deuxième valeur (peep2) qu'adopte la pression de fin d'expiration (PEEP) lors de la respiration artificielle, la compliance régionale résultant respectivement à cette valeur (peep1, peep2) des poumons dans cette zone pulmonaire (RegA, RegB).

**8.** Produit de programme informatique qui peut être exécuté sur un appareil de commande (16), et

lors de l'exécution sur l'appareil de commande (16), si l'appareil de commande (16) reçoit

- des signaux d'un dispositif de mesure EIT (17) pour mesurer une mesure de la variation de volume d'une zone (RegA, RegB) des poumons d'un patient (P),
- des signaux d'un capteur pneumatique de pression des voies respiratoires (3) pour mesurer une mesure de la pression variable dans le temps ($P_{aw}$) au niveau des voies respiratoires du patient (P), et
- des signaux d'un capteur pneumatique de pression de l'oesophage (10) pour mesurer une mesure de la pression variable dans le temps ($P_{es}$) dans l'œsophage (Sp) du patient (P),

fait que l'appareil de commande (16) exécute un procédé de détermination automatique d'une mesure de la compliance régionale respective {$C_{Lung}$[RegA], $C_{Lung}$[RegB]} des poumons d'un patient (P) dans plusieurs zones différentes (RegA, RegB) des poumons,
dans lequel le produit de programme informatique fait que lors de l'exécution du procédé les étapes suivantes sont exécutées

- le dispositif de mesure EIT (17) mesure au moyen de la tomographie par impédance électrique (EIT) une mesure de variation de volume de la zone pulmonaire (RegA, RegB),
- en utilisant des signaux du dispositif de mesure EIT (17), une mesure de la différence entre le volume de fin d'inspiration et le volume de fin d'expiration de la zone pulmonaire est déterminée, et
- comme une mesure de la compliance régionale {$C_{Lung}$[RegA], $C_{Lung}$[RegB]} de la zone pulmonaire, le quotient de la différence de volume pour cette zone et de la différence de pression présente au niveau des poumons ($\Delta$Ptp) est calculé,

dans lequel le produit de programme informatique fait que lors de l'exécution du procédé, les étapes suivantes sont exécutées

- la pression transpulmonaire de fin d'inspiration (EIPtp) est déterminée comme la différence entre la pression des voies respiratoires de fin d'inspiration (EIP) et la pression de l'œsophage de fin d'inspiration (EIPes), et
- la pression transpulmonaire de fin d'expiration (EEPtp) est déterminée comme la différence entre la pression des voies respiratoires de fin d'expiration (PEEP) et la pression de l'œsophage de fin d'expiration (EEPes).

FIG. 1

EP 3 957 241 B1

FIG. 2

EP 3 957 241 B1

FIG. 3

EP 3 957 241 B1

FIG. 4

**FIG. 5**

FIG. 6

EP 3 957 241 B1

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 102005031752 B4
- EP 2397074 B1

**In der Beschreibung aufgeführte Nicht-Patentliteratur**

- **ZHANQI ZHAO.** Positive End-Expiratory Pressure Titration with Electrical Impedance Tomography and Pressure-Volume Curve in Severe Acute Respiratory Distress Syndrome. *Annals of Intensive Care,* Dezember 2019, vol. 9 (1, https://doi.org/10.1186/s13613-019-0484-0
- **OLA STENQVIST ; PER PERSSON ; STEFAN LUNDIN.** Can We Estimate Transpulmonary Pressure without an Esophageal Balloon?-Yes. *Annals of Translational Medicine,* Oktober 2018, vol. 6 (19), 392, https://doi.org/10.21037/atm.2018.06.05
- **GIACOMO BELLANI.** Plateau and Driving Pressure in the Presence of Spontaneous Breathing. *Intensive Care Medicine,* Januar 2019, vol. 45 (1), 97-98, https://doi.org/10.1007/s00134-018-5311-9
- **DANIEL TALMOR.** Mechanical Ventilation Guided by Esophageal Pressure in Acute Lung Injury. *New England Journal of Medicine,* 13. November 2008, vol. 359 (20), 2095-2104, https://doi.org/10.1056/NEJMoa0708638
- **JEREMY R. BEITLER.** Effect of Titrating Positive End-Expiratory Pressure (PEEP) With an Esophageal Pressure-Guided Strategy vs. an Empirical High PEEP-FIO2 Strategy on Death and Days Free From Mechanical Ventilation Among Patients With Acute Respiratory Distress Syndrome: A Randomized Clinical Trial. *JAMA,* 18. Februar 2019, https://doi.org/10.1001/jama.2019.0555
- **EDUARDO L. V. COSTA.** Bedside Estimation of Recruitable Alveolar Collapse and Hyperdistension by Electrical Impedance Tomography. *Intensive Care Medicine,* Juni 2009, vol. 35 (6), 1132-37, https://doi.org/10.1007/s00134-009-1447-y